(19) 

Europäisches
Patentamt
European
Patent Office
Office européen
des brevets

(11) **EP 4 759 828 A1**

(12) **EUROPEAN PATENT APPLICATION**
published in accordance with Art. 153(4) EPC

(43) Date of publication:
**17.06.2026 Bulletin 2026/25**

(21) Application number: **24853689.8**

(22) Date of filing: **09.08.2024**

(51) International Patent Classification (IPC):
**C07K 16/24** (2006.01)     **C12N 15/13** (2006.01)
**A61K 39/395** (2006.01)    **A61P 37/00** (2006.01)

(52) Cooperative Patent Classification (CPC):
**A61K 39/395; A61P 37/00; C07K 16/00;
C07K 16/24**

(86) International application number:
**PCT/CN2024/110996**

(87) International publication number:
**WO 2025/036280 (20.02.2025 Gazette 2025/08)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **11.08.2023 CN 202311010530
06.08.2024 CN 202411069268**

(71) Applicant: **Innovent Biologics (Suzhou) Co., Ltd.
Suzhou, Jiangsu 215123 (CN)**

(72) Inventors:
• **CAI, Chenghang
Suzhou, Jiangsu 215123 (CN)**
• **QIAN, Lei
Suzhou, Jiangsu 215123 (CN)**

(74) Representative: **Sagittarius IP
Marlow International
Parkway
Marlow SL7 1YL (GB)**

Remarks:
The complete document including Reference
Table(s) and the Sequence Listing(s) can be
downloaded from the EPO website

(54) **METHOD FOR TREATING PLAQUE PSORIASIS WHEREIN SUBJECT PREVIOUSLY RECEIVING BIOLOGIC THERAPY FOR PLAQUE PSORIASIS SWITCHES TO USING ANTI-IL23P19 ANTIBODY**

(57)     The present invention relates to a method for treating plaque psoriasis in a subject previously treated with biological agents for plaque psoriasis, comprising administering to the subject an effective amount of an anti-IL23p19 subunit antibody, and to use of the anti-IL23p19 antibody in treating plaque psoriasis in a subject previously treated with biological agents.

EP 4 759 828 A1

**Description**

**FIELD OF THE INVENTION**

The present invention relates to use of an anti-IL23p19 antibody in treating a subject with plaque psoriasis previously treated with biological agents, and also relates to a method for treating a subject with plaque psoriasis previously treated with biological agents, comprising administering to the subject an effective amount of an anti-IL23p19 antibody.

**Background of the Invention**

IL-23 is a heterodimeric cytokine of IL-12 family members, consisting of a p40 subunit and a p19 subunit. IL-23 activates signaling pathways by binding to the IL-23 receptor (IL-23R) and the $\beta$1 subunit of the IL-12 receptor (IL-12R$\beta$1). Although IL-23 is structurally closely related to IL-12, these two cytokines have different biological functions, with IL-12 and IL-23 driving Th1 and Th17 cellular responses, respectively. IL-23 can independently induce naive CD4$^+$ T cells to differentiate into Th17 cells, or do so with the assistance of other cytokines such as transforming growth factor (TGF)-$\beta$, IL-6 or IL-1$\beta$; additionally, it can promote the proliferation, differentiation and maintenance of Th17 cells and innate immune cells. Th17 cell stimulation produces cytokines including IL-17, IL-22, tumor necrosis factor (TNF)-$\alpha$, and granulocyte-macrophage-colony stimulating factor (GM-CSF), which stimulate local tissue inflammation and other immune mediators through a wide range of immune-mediated inflammatory states. Chronic inflammation resulting from dysregulation of IL-23/Th17/IL-17 responses is the pathophysiological basis for a number of autoimmune diseases including psoriasis, ulcerative colitis, Crohn's disease, rheumatoid arthritis, multiple sclerosis and asthma.

Psoriasis is a chronic, recurrent, inflammatory, and systemic disease that is genetically and environmentally mediated and immune- mediated, and can occur at all ages without gender differences. The typical clinical manifestations are scaly erythema or plaques, localized or widespread, non-infectious, difficult to treat, and often lifelong. Psoriasis can be divided into vulgaris psoriasis (including guttate psoriasis and plaque psoriasis), pustular psoriasis, erythrodermic psoriasis, and arthropathic psoriasis.

At present, in China, the main systemic drug therapies for psoriasis include Methotrexate (MTX), Cyclosporine A, retinoids, and biological agents. In recent years, monoclonal antibody biological agents against cellular inflammatory factors have been successively used in the treatment of severe psoriasis with significant joint symptoms due to poor response to traditional systemic drugs and serious impact on quality of life, including tumor necrosis factor (TNF)-$\alpha$ antagonists (etanercept, infliximab, and adalimumab), IL-12/23 antagonists (ustekinumab), and IL-17A antagonists (secukinumab). Guselkumab (Johnson & Johnson), Risankizumab (Abbrev), and Tildrakizumab (Merck Sharp & dohme), which are humanized IgG1 monoclonal antibodies developed in recent years and bind to the IL-23p19 subunit, have shown significant efficacy in the treatment of plaque psoriasis and have been used in a number of countries to treat moderate to severe plaque psoriasis.

For biological agents, after a certain period of treatment, some patients experience loss of response, i.e., symptoms recur. At this time, new treatment means need to be introduced to effectively treat the patients with loss of response. At present, the utilization rate of biological agents for psoriasis patients in China is still very low, and there is still a huge demand for effective, safe, and low-cost domestic biological agents. In addition, for patients whose symptoms have been relieved by existing biological agents, switching to a treatment that is simpler and easier to administer and can maintain symptom relief will increase patient compliance and be more beneficial to patients.

**SUMMARY OF THE INVENTION**

The objective of the present invention is to provide a method for switching to recombinant anti-interleukin IL-23p19 antibody for the treatment of plaque psoriasis in subjects who have previously received biological agents for the treatment of plaque psoriasis, so as to meet the unmet needs in the art.

**Uses**

The present invention provides use of a recombinant anti-interleukin IL23p19 antibody in the treatment of plaque psoriasis in subjects who have previously received a biological agent therapy for plaque psoriasis.

In a first aspect, the present invention relates to use of a recombinant anti-interleukin-IL23p19 antibody in the manufacture of a medicament for treating plaque psoriasis in a subject.

In a second aspect, the present invention relates to a recombinant anti-interleukin-IL23p19 antibody for use in treating plaque psoriasis in a subject.

For the first and second aspects, further embodiments thereof are as follows:

In some embodiments, the recombinant anti-interleukin IL23p19 antibody comprises a heavy chain variable region and a light chain variable region, wherein CDR1 of the heavy chain variable region comprises or consists of an amino acid sequence of SEQ ID NO. 1, CDR2 of the heavy chain variable region comprises or consists of an amino acid sequence of SEQ ID NO. 2, and CDR3 of the heavy chain variable region comprises or consists of an amino acid sequence of SEQ ID NO. 3; CDR1 of the light chain variable region comprises or consists of an amino acid sequence of SEQ ID NO. 4, CDR2 of the light chain variable region comprises or consists of an amino acid sequence of SEQ ID NO. 5, and CDR3 of the light chain variable region comprises or consists of an amino acid sequence of SEQ ID NO. 6.

In some embodiments, the recombinant anti-interleukin IL23p19 antibody comprises a heavy chain variable region and a light chain variable region, wherein the heavy chain variable region comprises the sequence of SEQ ID NO: 7 or a sequence having at least 90%, 95%, 98%, or 99% identity thereto, and the light chain variable region comprises the sequence of SEQ ID NO: 8 or a sequence having at least 90%, 95%, 98%, or 99% identity thereto.

In some embodiments, the recombinant anti-interleukin IL23p19 antibody is an IgG1 antibody, preferably comprising a heavy chain sequence of SEQ ID NO: 9 or of a sequence having at least 90%, 95%, 98%, or 99% identity to SEQ ID NO: 9 , and a light chain sequence of SEQ ID NO: 10 or of a sequence having at least 90%, 95%, 98%, or 99% identity SEQ ID NO: 10.

In some embodiments, the recombinant anti-interleukin IL23p19 antibody comprises a heavy chain as shown in SEQ ID NO: 9 and a light chain as shown in SEQ ID NO: 10.

In some embodiments, the subject has previously received a biological agent for the treatment of plaque psoriasis. In some embodiments, the subject is switched to the antibody of the present invention for the treatment of plaque psoriasis after previously receiving a biological agent for the treatment of plaque psoriasis.

In some embodiments, the subject may be one who is regularly administrated biological agents according to the prescribed dose (for at least 4 months) or one who is not regularly administrated biological agents according to the prescribed dose (at least 4 months from the last dose of biological agents to the first dose of biological agents ) prior to screening.

In some embodiments, the subject is unresponsive or poorly responsive to previous biologic therapy. In some embodiments, the subject is being treated with a biological agent but has lost response or has an inadequate response. In some embodiments, subjects who have lost response or exhibited poor response to previous biologic therapy have an sPGA score of $\geq 2$, or a cumulative involved body surface area of skin lesions of $\geq 3\%$, or a PASI improvement of less than 75% after biologic therapy administration.

In some embodiments, for subjects who have lost response or exhibited poor response to previous biologic therapy, administration of the antibody of the present invention comprises a treatment phase and a maintenance phase. In some embodiments, in the treatment phase, the administration frequency of the antibody of the present invention is once daily, twice weekly, once weekly, once every two weeks, once every four weeks, once every five weeks, once every six weeks, once every seven weeks, once every eight weeks, or longer. The administration cycle of the antibody of the present invention may be one week, two weeks, three weeks, one month, two months, three months or longer, and the interval between each administration cycle may be the same or different. In one preferred embodiment, the treatment phase comprises administering the anti-IL-23p19 antibody at weeks 0, 4, and 8. In some embodiments, in the maintenance phase, the administration frequency of the antibody of the present invention is once every four weeks, once every eight weeks, once every twelve weeks, once every sixteen weeks, once every twenty weeks, or longer. In some embodiments, the maintenance phase lasts for at least 24, 28, 32, 36, 40, 44, 48 weeks or longer. In one preferred embodiment, in the maintenance phase, administration is performed once every 12 weeks until the last dose at week 32. In some embodiments, the anti-IL-23p19 antibody is administered at weeks 0, 4, and 8, respectively, and every 12 weeks thereafter to subjects who have lost response or exhibited poor response to previous biological therapy. In some embodiments, the anti-IL-23p19 antibody is administered at weeks 0, 4, and 8, respectively, and every 12 weeks thereafter until the last dose at week 32 to subjects who have lost response or have exhibited poor response to previous biological therapy.

In some embodiments, the dosage for each administration is selected from 100 mg-1000 mg, preferably 150 mg-800 mg, e.g., 100 mg, 110 mg, 120 mg, 130 mg, 140 mg, 150 mg, 160 mg, 170 mg, 180 mg, 190 mg, 200 mg, 210 mg, 220 mg, 230 mg, 240 mg, 250 mg, 260 mg, 270 mg, 280 mg, 290 mg, 300 mg, 350 mg, 400 mg, 450 mg, 500 mg, 550 mg, 600 mg, 650 mg, 700 mg, 750 mg, 800 mg, 850 mg, 900 mg, 950 mg, or 1000 mg. In some embodiments, the dosage for each administration of the anti-IL-23P19 antibody is of 150 mg, 160 mg, 170 mg, 180 mg, 190 mg, 200 mg, 210 mg, 220 mg, 230 mg, 240 mg, or 250 mg. In a preferred embodiment, the dosage for each administration is 200 mg.

In some embodiments, for subjects who have lost response or exhibited poor response to previous biological therapy, 200 mg of the anti-IL-23p19 antibody is administered at weeks 0, 4, and 8, respectively, and 200 mg of the anti-IL-23p19 antibody is administered once every 12 weeks thereafter. In some embodiments, for subjects who have lost response or exhibited poor response to previous biological therapy, 200 mg of the anti-IL-23p19 antibody is administered at weeks 0, 4, and 8, respectively, and 200 mg of the anti-IL-23p19 antibody is administered every 12 weeks thereafter until the last dose at week 32.

In some embodiments, the subject is a responder to a previous biologic agent. In some embodiments, the subject is being treated with a biological agent and still responds. In some embodiments, the subject has a sPGA score of 0 or 1 and a cumulative body surface area of skin lesions of < 3%, or achieves a PASI improvement of 75% or more after using a biological agent.

In some embodiments, for subjects who have responded to previous biological treatment, the administration frequency of the antibody of the present invention is once a day, twice a week, once a week, once every two weeks, once every four weeks, once every five weeks, once every six weeks, once every seven weeks, once every eight weeks, or longer. The administration cycle of the antibody of the present invention may be one week, two weeks, three weeks, one month, two months, three months or longer, and the interval of each administration cycle may be the same or different. In one preferred embodiment, the anti-IL-23p19 antibody is administered at week 0. In some embodiments, following administration at week 0, a dosing interval of once every four weeks, once every eight weeks, once every twelve weeks, once every sixteen weeks, once every twenty weeks, or longer is maintained. In some embodiments, the maintenance administration lasts for at least 24, 28, 32, 36, 40, 44, 48 weeks or longer. In one preferred embodiment, the maintenance administration is performed once every 12 weeks thereafter. In a preferred embodiment, an anti-IL-23p19 antibody is administered at Week 0 and every 12 weeks thereafter to subjects who have responded to previous biologic therapy. In one embodiment, an anti-IL-23p19 antibody is administered at Week 0 and every 12 weeks thereafter until the last dose at Week 36 to a subject who is responsive to previous biologic therapy.

In some embodiments, each administration dose is selected from 100 mg-1000 mg, preferably 150 mg-800 mg, e.g., 100 mg, 110 mg, 120 mg, 130 mg, 140 mg, 150 mg, 160 mg, 170 mg, 180 mg, 190 mg, 200 mg, 210 mg, 220 mg, 230 mg, 240 mg, 250 mg, 260 mg, 270 mg, 280 mg, 290 mg, 300 mg, 350 mg, 400 mg, 450 mg, 500 mg, 550 mg, 600 mg, 650 mg, 700 mg, 750 mg, 800 mg, 850 mg, 900 mg, 950 mg, or 1000 mg. In some embodiments, the anti-IL-23P19 antibody is administered at a dose of 150 mg, 160 mg, 170 mg, 180 mg, 190 mg, 200 mg, 210 mg, 220 mg, 230 mg, 240 mg, or 250 mg. In a preferred embodiment, the dosage for each administration is 200 mg.

In some embodiments, for a subject who is responsive to previous biologic therapy, 200 mg of the anti-IL-23p19 antibody is administered at Week 0 and 200 mg of the anti-IL-23p19 antibody is administered every 12 weeks thereafter. In some embodiments, for subjects who are responsive to previous biologic therapy, 200 mg of the anti-IL-23p19 antibody is administered at Week 0, and 200 mg of the anti-IL-23p19 antibody is administered every 12 weeks thereafter until the last dose at Week 36.

In some embodiments, the biological agent includes a tumor necrosis factor $\alpha$ (TNF-$\alpha$) inhibitor/antagonist drug and/or an IL-17A inhibitor/antagonist drug.

In some embodiments, the TNF-$\alpha$ inhibitor/antagonist drug includes etanercept (Enbrel), infliximab (Remicade), adalimumab (Humira), golimumab (Simponi), and cetuximab (Cimzia).

In some embodiments, the IL-17A inhibitor/antagonist drug includes secukinumab (Cosentyx) and ixekizumab (Taltz).

In some embodiments, the anti-IL-23p19 antibody of the present invention is formulated for administration in the form of a liquid pharmaceutical composition. Useful carriers and solvents include water, Ringer's solution, phosphate-buffered saline, isotonic sodium chloride solution, and the like. In addition, sterile, fixed oils may also be employed as a solvent or suspending medium where appropriate. Any mixed non-volatile mineral oil or non-mineral oil may be utilized for this purpose, including synthetic mono- or diglycerides. In addition, fatty acids such as oleic acid may also be used in the preparation of injectables.

In some embodiments, the pharmaceutical composition comprising the anti-IL-23p19 antibody of the present invention is a solution for injection or a dry powder formulation. For example, the composition is a lyophilized powder, which can be reconstituted into an injection solution in a pharmaceutically acceptable liquid carrier. The pharmaceutically acceptable liquid carrier may be, for example, sterile water, Ringer's solution, phosphate-buffered saline, isotonic sodium chloride solution, and the like.

In some embodiments, the anti-IL-23p19 antibody of the present invention is administered locally.

In some embodiments, the anti-IL-23p19 antibody of the present invention is administered by subcutaneous injection.

In some embodiments, the subject does not experience a serious adverse event after administration.

In some embodiments, after administration, the incidence of adverse events in the subject is comparable to that in a subject receiving placebo.

In some embodiments, the subject is a human.

In some embodiments, the administration of the anti-IL-23p19 antibody of the present invention to a subject can improve psoriatic skin lesions and quality of life in such subjects who have lost response or have responded poorly to previous biological treatments. In some embodiments, the administration of the anti-IL-23p19 antibody of the present invention to a subject can enable such subjects who are responsive to previous biological treatment to continue to maintain a responsive state or improve response (e.g., improve skin lesions) to the treatment and maintain the current quality of life or improve the current quality of life.

In one preferred embodiment, the anti-IL-23p19 antibody of the present invention can achieve one or more of the following effects in a subject:

The maximum dosing interval for previous IL-17 and TNF class drugs was once every four weeks, which entails relatively frequent administration and is prone to cause inconvenience to patients. In contrast, when the anti-IL-23p19 antibody of the present invention is administered via the dosing regimen provided herein, the reduced administration frequency not only renders the dosing procedure simpler and more convenient, but also improves patients' compliance and quality of life.

Study data showed that among subjects with baseline clinical response who switched to anti-IL-23p19 antibody therapy, 85.3% of the subjects still maintained clinical response at Week 16 (with the cumulative body surface area involved in skin lesions < 3% and a static Physician's Global Assessment [sPGA] score of 0 or 1), and the response rate remained stable at approximately 85% from Week 16 to the end of the 36-week observation period. In contrast, among subjects with baseline clinical non-response who switched to the aforementioned antibody therapy, some subjects began to exhibit clinical response; the response rate gradually increased to 52.1% by Week 16 and was maintained at a similar level after Week 16.

## Methods of treatment

In a third aspect, the present invention relates to a method for treating plaque psoriasis in a subject, comprising administering to the subject an effective amount of a recombinant anti-interleukin IL23p19 antibody.

In a fourth aspect, the present invention provides a method for treating plaque psoriasis in subjects previously administered with biological agents, wherein the subjects are switched to receive a recombinant anti-interleukin-23p19 antibody..

For the third and fourth aspects, further embodments thereof are as follows:

In some embodiments, the anti-IL23p19 antibody comprises a heavy chain variable region and a light chain variable region, wherein the amino acid sequence of the CDR1 of the heavy chain variable region comprises or consists of SEQ ID NO. 1, the amino acid sequence of the CDR2 of the heavy chain variable region comprises or consists of SEQ ID NO. 2, and the amino acid sequence of the CDR3 of the heavy chain variable region comprises or consists of SEQ ID NO. 3; the amino acid sequence of the CDR1 of the light chain variable region of the antibody comprises or consists of SEQ ID NO. 4, the amino acid sequence of the CDR2 of the light chain variable region comprises or consists of SEQ ID NO. 5, and the amino acid sequence of the CDR3 of the light chain variable region comprises or consists of SEQ ID NO. 6.

In some embodiments, the recombinant anti-interleukin IL23p19 antibody comprises a heavy chain variable region VH and a light chain variable region VL, wherein the heavy chain variable region comprises the sequence of SEQ ID NO: 7 or a sequence having at least 90%, 95%, 98%, or 99% identity thereto, and the light chain variable region comprises the sequence of SEQ ID NO: 8 or a sequence having at least 90%, 95%, 98%, or 99% identity thereto.

In some embodiments, the recombinant anti-interleukin IL23p19 antibody is an IgG1 antibody, preferably comprising a heavy chain sequence of SEQ ID NO: 9 or a sequence having at least 90%, 95%, 98%, or 99% identity thereto and a light chain sequence of SEQ ID NO: 10 or a sequence having at least 90%, 95%, 98%, or 99% identity thereto.

In some embodiments, the recombinant anti-interleukin IL23p19 antibody comprises a heavy chain set forth in SEQ ID NO: 9 and a light chain set forth in SEQ ID NO: 10.

In some embodiments, the subject has previously received a biological agent for the treatment of plaque psoriasis. In some embodiments, the subject is switched to the antibody of the present invention for the treatment of plaque psoriasis after previously receiving a biological agent for the treatment of plaque psoriasis.

In some embodiments, prior to screening, the subject may be one who regularly receives biological agents according to the prescribed dose (for at least 4 months) or one who does not regularly receive biological agents according to the prescribed dose (at least 4 months from the last dose of biological agents to the first dose of biological agents) .

In some embodiments, the method comprises administering an anti-IL-23p19 antibody to a subject previously treated with biological agents for plaque psoriasis.

In some embodiments, the subjects who have lost response or exhibited poor response to previous biological therapy. In some embodiments, the subject is being treated with a biological agent but has lost response or has an inadequate response. In some embodiments, subjects who have lost response to or exhibited poor response to previous biological agents have an sPGA score of $\geq 2$, a cumulative body surface area affected by skin lesions of $\geq 3\%$, or a PASI improvement of less than 75% following biological agents administration.

In some embodiments, for subjects who have lost response to or exhibited poor response to previous biological therapy, the method of the present invention comprises administering the antibody of the present invention in two phases, namely a treatment phase and a maintenance phase. In some embodiments, in the treatment phase, the administration frequency of the antibody of the present invention is once daily, twice weekly, once weekly, once every two weeks, once every four weeks, once every five weeks, once every six weeks, once every seven weeks, once every eight weeks, or longer. The administration cycle of the antibody of the present invention may be one week, two weeks, three weeks, one month, two months, three months or longer, and the interval of each administration cycle may be the same or different. In one preferred embodiment, the treatment phase comprises administering the anti-IL-23p19 antibody at weeks 0, 4, and 8.

In some embodiments, in the maintenance phase, the administration frequency of the antibody of the present invention is once every four weeks, once every eight weeks, once every twelve weeks, once every sixteen weeks, once every twenty weeks, or longer. In some embodiments, the maintenance phase lasts for at least 24, 28, 32, 36, 40, 44, 48 weeks or longer. In one preferred embodiment, the maintenance phase is performed once every 12 weeks until the last dose at week 32. In some embodiments, the anti-IL-23p19 antibody is administered at weeks 0, 4, and 8, respectively, and every 12 weeks thereafter to a subject who have lost response or exhibited poor response to previous biological therapy. In some embodiments, the anti-IL-23p19 antibody is administered at weeks 0, 4, and 8, respectively, and every 12 weeks thereafter until the last dose at week 32 to subjects who have lost response or exhibited poor response to previous biological therapy.

In some embodiments, the dosage for each administration is 100 mg to 1000 mg, preferably 150 mg to 800 mg, e.g., 100 mg, 110 mg, 120 mg, 130 mg, 140 mg, 150 mg, 160 mg, 170 mg, 180 mg, 190 mg, 200 mg, 210 mg, 220 mg, 230 mg, 240 mg, 250 mg, 260 mg, 270 mg, 280 mg, 290 mg, 300 mg, 350 mg, 400 mg, 450 mg, 500 mg, 550 mg, 600 mg, 650 mg, 700 mg, 750 mg, 800 mg, 850 mg, 900 mg, 950 mg, or 1000 mg. In some embodiments, the dosage for each administration is 150 mg, 160 mg, 170 mg, 180 mg, 190 mg, 200 mg, 210 mg, 220 mg, 230 mg, 240 mg, or 250 mg anti-IL-23P19 antibody. In a preferred embodiment, the dosage for each administration is 200 mg.

In some embodiments, for subjects who have lost response to or exhibited poor response to previous biological therapy, 200 mg of the anti-IL-23p19 antibody is administered at weeks 0, 4, and 8, respectively, and 200 mg of the anti-IL-23p19 antibody is administered once every 12 weeks thereafter. In some embodiments, for subjects who have lost response to or exhibited poor response to previous biological therapy, 200 mg of the anti-IL-23p19 antibody is administered at weeks 0, 4, and 8, respectively, and 200 mg of the anti-IL-23p19 antibody is administered every 12 weeks thereafter until the last dose at week 32.

In some embodiments, the subject is a responder to a previous biologic therapy. In some embodiments, the subject is being treated with a biological agent and still responds. In some embodiments, the subject has a sPGA score of 0 or 1 and a cumulative body surface area of skin lesions of < 3%, or achieves a PASI improvement of 75% or more after using a biological agent.

In some embodiments, for subjects who have responded to previous biological treatment, the administration frequency of the antibody of the present invention is once every week, once every two weeks, once every four weeks, once every five weeks, once every six weeks, once every seven weeks, once every eight weeks, or once every twelve weeks, once every sixteen weeks, once every twenty weeks, or longer. The administration cycle of the antibody of the present invention may be one week, two weeks, three weeks, one month, two months, three months or longer, and the interval of each administration cycle may be the same or different. In one preferred embodiment, the anti-IL-23p19 antibody is administered at week 0. In some embodiments, following administration at Week 0, dosing is maintained once every 4 weeks, once every 8 weeks, once every 12 weeks, once every 16 weeks, once every 20 weeks, or at longer intervals. In some embodiments, the maintenance administration lasts for at least 24, 28, 32, 36, 40, 44, 48 weeks or longer. In a preferred embodiment, the maintenance administration is performed once every 12 weeks for at least 24, 28, 32, 36, 40, 44, 48 weeks or longer. In a preferred embodiment, an anti-IL-23p19 antibody is administered at Week 0 and every 12 weeks thereafter for at least 24, 28, 32, 36, 40, 44, 48 weeks or longer to a subject who is responsive to previous biologic therapy. In one embodiment, an anti-IL-23p19 antibody is administered at Week 0 and every 12 weeks thereafter until the last dose at Week 36 to a subject who is responsive to previous biologic therapy.

In some embodiments, the dosage for each administration is selected from 100 mg-1000 mg, preferably 150 mg-800 mg, e.g., 100 mg, 110 mg, 120 mg, 130 mg, 140 mg, 150 mg, 160 mg, 170 mg, 180 mg, 190 mg, 200 mg, 210 mg, 220 mg, 230 mg, 240 mg, 250 mg, 260 mg, 270 mg, 280 mg, 290 mg, 300 mg, 350 mg, 400 mg, 450 mg, 500 mg, 550 mg, 600 mg, 650 mg, 700 mg, 750 mg, 800 mg, 850 mg, 900 mg, 950 mg, or 1000 mg. In some embodiments, the dosage for each administration is of 150 mg, 160 mg, 170 mg, 180 mg, 190 mg, 200 mg, 210 mg, 220 mg, 230 mg, 240 mg, or 250 mg the anti-IL-23P19 antibody. In a preferred embodiment, the dosage for each administration is 200 mg.

In some embodiments, for a subject who is responsive to previous biologic therapy, 200 mg of the anti-IL-23p19 antibody is administered at Week 0 and 200 mg of the anti-IL-23p19 antibody is administered every 12 weeks thereafter. In some embodiments, for subjects who are responsive to previous biologic therapy, 200 mg of the anti-IL-23p19 antibody is administered at Week 0, and 200 mg of the anti-IL-23p19 antibody is administered every 12 weeks thereafter until the last dose at Week 36.

In some embodiments, the biological agent includes a tumor necrosis factor α inhibitor/antagonist drug and/or an IL-17A inhibitor/antagonist drug.

In some embodiments, the TNF-α inhibitor/antagonist drug includes etanercept, infliximab, adalimumab, golimumab, and trastuzumab.

In some embodiments, the IL-17A inhibitor/antagonist drug includes secukinumab and ixekizumab.

In some embodiments, the anti-IL-23p19 antibody is recombinantly expressed in HEK 293 cells or CHO cells.

In some embodiments, the anti-IL-23p19 antibody of the present invention is formulated for administration in the form of a liquid pharmaceutical composition. Useful carriers and solvents include water, Ringer's solution, phosphate-buffered saline, isotonic sodium chloride solution, and the like. In addition, sterile, fixed oils may also be employed as a solvent or suspending medium where appropriate. Any mixed non-volatile mineral oil or non-mineral oil may be utilized for this purpose, including synthetic mono- or diglycerides. In addition, fatty acids such as oleic acid may also be used in the preparation of injectables.

In some embodiments, the pharmaceutical composition comprising the anti-IL-23p19 antibody of the present invention is a solution for injection or a dry powder formulation. For example, the composition is a lyophilized powder, which can be reconstituted into an injection solution in a pharmaceutically acceptable liquid carrier. The pharmaceutically acceptable liquid carrier may be, for example, sterile water, Ringer's solution, phosphate-buffered saline, isotonic sodium chloride solution, and the like.

In some embodiments, the anti-IL-23p19 antibody or the pharmaceutical composition of the present invention is administered locally.

In some embodiments, the anti-IL-23p19 antibody or the pharmaceutical composition of the present invention is administered by subcutaneous injection.

In some embodiments of the above methods, the subject does not experience a serious adverse event after administration.

In some embodiments of the above methods, after administration, the incidence of adverse events in the subject is comparable to that in a subject receiving placebo.

In some embodiments of the above methods, the subject is a human.

The method of the present invention for treating plaque psoriasis in subjects previously treated with a biological agent, which involves switching the said subjects to a recombinant anti-interleukin-23p19 subunit antibody, can treat plaque psoriasis subjects who are being treated with a biological agent but have lost response to or exhibited poor response to previous biological therapy, so that the psoriasis lesions of such subjects are improved, and the quality of life of such subjects is improved; in addition, the method can also treat plaque psoriasis subjects who are receiving a biological agent and still responding to a biological agent, so that such subjects sustain to the treatment response or achieve an improved response (e.g., to improve the psoriasis skin lesions), as well as to maintain the current quality of life or improve the current quality of life.

Compared with similar products, the method of the present invention can include both psoriasis subjects who have lost response to biological agents and psoriasis subjects who have responded to biological agents. In addition, the actual clinical administration of biologics does not fully comply with the instructions for use. The method of the present invention exhibits certain therapeutic efficacy not only on subjects who follow the instructions for use and switch to the anti-IL-23p19 antibody of the present invention, but also on those who do not adhering to the instructions for use and switch to the antibody, thus covers a broader population of subjects.

In one preferred embodiment, the anti-IL-23p19 antibody of the present invention can achieve one or more of the following effects in a subject:

The maximum dosing interval of previous IL-17 and TNF class drugs was once every four weeks, which entailed relatively frequent administration and thus tended to cause inconvenience to patients. In contrast, when the anti-IL-23p19 antibody of the present invention is administered in accordance with the dosing regimen provided herein, the reduced administration frequency not only simplifies the dosing procedure and enhances its convenience, but also improves patients' compliance and quality of life.

Study data showed that among subjects with baseline clinical response who switched to anti-IL-23p19 antibody therapy, 85.3% still maintained clinical response at Week 16 (with cumulative body surface area affected by skin lesions < 3% and a static Physician's Global Assessment [sPGA] score of 0 or 1), and the response rate remained stable at approximately 85% from Week 16 to the end of the 36-week observation period. Among subjects with baseline clinical non-response who switched to the aforementioned antibody therapy, some subjects began to exhibit clinical response; the response rate gradually increased to 52.1% by Week 16 and was maintained at a similar level after Week 16.

It should be understood that any technical solution derived from the arbitrary combination of any technical feature described in the first and second aspects above with any technical feature described in the third and fourth aspects is also encompassed in the present invention.

**Single Pharmaceutical Dosage Unit or Pharmaceutical Kit**

In a fifth aspect, the present invention relates to a single pharmaceutical dosage unit comprising the anti-IL23p19 antibody of the present invention.

In some embodiments, the recombinant anti-interleukin IL23p19 antibody comprises a heavy chain variable region and a light chain variable region, wherein an amino acid sequence of a CDR1 of the heavy chain variable region comprises or consists of SEQ ID NO. 1, an amino acid sequence of a CDR2 of the heavy chain variable region comprises or consists of SEQ ID NO. 2, and an amino acid sequence of a CDR3 of the heavy chain variable region comprises or consists of SEQ ID NO. 3; an amino acid sequence of a CDR1 of the light chain variable region comprises or consists of SEQ ID NO. 4, an amino acid sequence of a CDR2 of the light chain variable region comprises or consists of SEQ ID NO. 5, and an amino acid sequence of a CDR3 of the light chain variable region comprises or consists of SEQ ID NO. 6.

In some embodiments, the recombinant anti-interleukin IL23p19 antibody comprises a heavy chain variable region and a light chain variable region, wherein the heavy chain variable region comprises the sequence of SEQ ID NO: 7 or a sequence having at least 90%, 95%, 98%, or 99% identity thereto, and the light chain variable region comprises the sequence of SEQ ID NO: 8 or a sequence having at least 90%, 95%, 98%, or 99% identity thereto.

In some embodiments, the recombinant anti-interleukin IL23p19 antibody is an IgG1 antibody, preferably comprising a heavy chain sequence of SEQ ID NO: 9 or a sequence having at least 90%, 95%, 98%, or 99% identity thereto and a light chain sequence of SEQ ID NO: 10 or a sequence having at least 90%, 95%, 98%, or 99% identity thereto.

In some embodiments, the recombinant anti-interleukin IL23p19 antibody comprises a heavy chain set forth in SEQ ID NO: 9 and a light chain set forth in SEQ ID NO: 10.

In some embodiments, the single pharmaceutical dosage unit comprises the following dosage of the anti - IL - 23p19 antibody: a fixed dose of 10-1000 mg, preferably 50-800 mg, more preferably 50-500 mg, e.g., 50 mg, 60 mg, 70 mg, 80 mg, 90 mg, 100 mg, 110 mg, 120 mg, 130 mg, 140 mg, 150 mg, 160 mg, 170 mg, 180 mg, 190 mg, 200 mg, 210 mg, 220 mg, 230 mg, 240 mg, 250 mg, 260 mg, 270 mg, 280 mg, 290 mg, 300 mg, 350 mg, 400 mg, 450 mg, 500 mg, 550 mg, 600 mg, 650 mg, 700 mg, 750 mg, 800 mg, 850 mg, 900 mg, 950 mg, or 1000 mg.

In some embodiments, the single pharmaceutical dosage unit is a single pharmaceutical dosage unit packaged in a prefilled automated injection pen.

In a sixth aspect, the present invention relates to a pharmaceutical kit, comprising the anti-IL23p19 antibody of the present invention according to any one of the embodiments of the single pharmaceutical dosage unit described above.

In a seventh aspect, the present invention relates to use of the single pharmaceutical dosage unit as described above or the pharmaceutical kit as described above in the manufacture of a medicament for treating plaque psoriasis.

In some embodiments, provided is the single pharmaceutical dosage unit described above or the pharmaceutical kit described above for use in treating plaque psoriasis.

It should be understood that technical solutions obtained from any combination of any of the technical features described in the fifth to seventh aspects above and any of the technical features of the technical solutions described in the first to fourth aspects are also included in the present invention.

Other embodiments of the present invention will be apparent from and elucidated with reference to the following detailed description.

## DETAILED DESCRIPTION OF THE INVENTION

Before describing the present invention in detail, it is to be understood that this invention is not limited to the particular methods and experimental conditions described herein, as such methods and conditions may vary. Additionally, the terminology used herein is for the purpose of describing particular embodiments only and is not intended to be limiting.

## DEFINITIONS

Unless defined otherwise, all technical and scientific terms used herein have the same meaning as commonly understood by one of ordinary skill in the art. For the purposes of the present invention, the following terms are defined below.

The term "about", when used in conjunction with a numerical value, is intended to encompass the numerical values in a range from a lower limit less than the specified numerical value by 5% to an upper limit greater than the specified numerical value by 5%.

The term "and/or", when used to connect two or more options, should be understood to refer to any one of the options or any two or more of the options.

As used herein, the term "comprise" or "include" is intended to mean that the described elements, integers or steps are included, but not to the exclusion of any other elements, integers or steps. As used herein, the term "comprise" or "include", unless otherwise specified, also encompasses the situation where the entirety consists of the described elements, integers or steps. For example, when referring to an antibody variable region "comprising" a particular sequence, it is also intended to encompass an antibody variable region consisting of that particular sequence.

The p19 subunit of IL-23 (also referred to herein as "IL-23p19" and "p19 subunit") is a polypeptide of 189 amino acids containing a leader sequence of 21 amino acids (Oppmann et al., Immunity 13:715 (2000), SEQ ID NO: 181) and comprising four packed alpha helices designated A, B, C and D, with an up-up-down-down topology. The four helices are connected by three polypeptide loops. The A-B and C-D loops are made relatively long because they connect parallel helices. A short B-C loop connects the antiparallel B and C helices. The p19 subunit of IL-23 is a member of the IL-6 family of helical cytokines. This cytokine family binds to its cognate receptors via 3 conserved epitopes (sites I, II, and III; bravo and Heath (2000) EMBO J. 19:2399-2411). The p19 subunit interacts with three cytokine receptor subunits to form a competent signal transduction complex. When expressed in a cell, the p19 subunit first forms a complex with the p40 subunit, which is shared by the p19 subunit and IL-12. The p19p40 complex is secreted from cells as a heterodimeric protein and is referred to as IL-23. In one embodiment, the IL-23p19 of the present invention is derived from human (NCBI:AAG37232) or cynomolgus monkey (NCBI:AEY84629).

The term "anti-IL-23p19 antibody", "anti-IL-23p19", "recombinant anti-interleukin IL23p19 antibody ", "IL-23p19 antibody", or "antibody that binds to IL-23p19" as used herein refers to an antibody that is capable of binding to a (human or cynomolgus monkey) IL-23p19 subunit or a fragment thereof with sufficient affinity such that the antibody can be used as a diagnostic and/or therapeutic agent in targeting (human or cynomolgus monkey) IL-23p19.

As used herein, the term "antibody" is used in the broadest sense, refers to a protein comprising an antigen-binding site, and encompasses natural and artificial antibodies of various structures, including but not limited to intact antibodies and antigen-binding fragments of antibodies.

The terms "whole antibody", "full-length antibody", "complete antibody" and "intact antibody" are used interchangeably herein to refer to a glycoprotein comprising at least two heavy (H) chains and two light (L) chains interconnected by disulfide bonds. Each heavy chain consists of a heavy chain variable region (abbreviated herein as VH) and a heavy chain constant region. The heavy chain constant region consists of 3 domains, CH1, CH2 and CH3. Each light chain consists of a light chain variable region (abbreviated herein as VL) and a light chain constant region. The light chain constant region consists of one domain, CL. The VH and VL regions can be further subdivided into regions of hypervariability, termed complementarity determining regions (CDR), interspersed with regions that are more conserved, termed framework regions (FR). Each VH and VL is composed of three CDRs and four FRs arranged from amino-terminus to carboxy-terminus in the following order: FR1, CDR1, FR2, CDR2, FR3, CDR3, FR4. The constant region is not directly involved in the binding of an antibody to an antigen, but exhibits various effector functions.

A "complementarity determining region" or "CDR region" or "CDR" is a region in an antibody variable domain that is highly variable in sequence and forms structurally defined loops ("hypervariable loops") and/or contains antigen-contacting residues ("antigen-contacting sites"). The CDRs are primarily responsible for binding to antigenic epitopes. The CDRs of the heavy and light chains are generally referred to as CDR1, CDR2, and CDR3, and are numbered sequentially from the N-terminus. The CDRs located in the heavy chain variable domain of an antibody are referred to as HCDR1, HCDR2 and HCDR3, and the CDRs located in the light chain variable domain of an antibody are referred to as LCDR1, LCDR2 and LCDR3.

In a given amino acid sequence of a light chain variable region or a heavy chain variable region, the exact amino acid sequence boundary of each CDR can be determined using any one or a combination of many well-known antibody CDR assignment systems including, for example, Chothia based on the three-dimensional structure of antibodies and the topology of CDR loops (Chothia et al. (1989) Nature 342:877-883, Al-Lazikani et al., "Standard conformations for the canonical structures of immunoglobulins", Journal of Molecular Biology, 273, 927-948 (1997)), Kabat based on antibody sequence variability (Kabat et al., Sequences of Proteins of Immunological Interest, 4th edition, U.S. Department of Health and Human Services, National Institutes of Health (1987)), AbM (University of Bath), Contact (University College London), International ImMunoGeneTics database (IMGT) (on the World Wide Web imgt.cines.fr/), and North CDR definition based on the affinity propagation clustering using a large number of crystal structures.

Unless otherwise stated, residue positions of an antibody variable region (including heavy chain variable region residues and light chain variable region residues) are numbered according to the Kabat numbering system (Kabat et al., Sequences of Proteins of Immunological Interest, 5th Ed. Public Health Service, National Institutes of Health, Bethesda, Md. (1991)).

In one embodiment, the boundaries of the CDRs of the antibody of the present invention are determined by AbM rules.

An "antibody fragment" refers to a molecule other than an intact antibody that comprises a portion of an intact antibody and binds to the antigen to which the intact antibody binds. Examples of antibody fragments include, but are not limited to, Fv, Fab, Fab', Fab'-SH, F(ab')2; diabodies; linear antibodies; single-chain antibodies (e.g., scFv); single-domain antibodies; bivalent or bispecific antibodies or fragments thereof; camelid antibodies; and bispecific or multispecific antibodies formed from antibody fragments.

"Treating" refers to slowing, interrupting, arresting, alleviating, stopping, reducing, or reversing the progression or severity of an existing symptom, disorder, condition, or disease.

The term "effective amount" refers to an amount or dosage of the formulation or antibody of the present invention that produces the desired effect in a treated patient after administration to the patient in a single or multiple doses. The effective amount can be readily determined by an attending physician as a person skilled in the art by considering a variety of factors as follows: species such as mammals; its size, age, and general health; the specific disease involved; the degree or severity of the disease; response in an individual patient; specific antibody administered; mode of administration; bioavailability characteristics of the administered formulation; selected administration regimen; and use of any concomitant therapy.

A "therapeutically effective amount" refers to an amount that, at the required dosage and for the required period of time, effectively achieves the desired therapeutic outcome. A therapeutically effective amount of the formulation, antibody or antibody fragment, or conjugate or composition thereof of the present invention may vary depending on a variety of factors such as the disease state, age, sex, and weight of the individual, and the ability of the antibody or antibody portion to elicit a desired response in the individual. The therapeutically effective amount is also such an amount that any toxic or detrimental effect of the formulation, antibody or antibody fragment, or conjugate or composition thereof is inferior to the therapeutically beneficial effect.

A "formulation" or "pharmaceutical composition" refers to a composition comprising at least one active ingredient and at least one inactive ingredient which is suitable for administration to animals, preferably mammals, including humans. A "liquid formulation" or "liquid composition" refers to a formulation in liquid form. The liquid composition of the present invention comprises (i) the antibody of the present invention; (ii) a buffer; and (iii) a vehicle. The composition of the formulation of the present invention may be as shown in the embodiments relating to the liquid pharmaceutical composition above. The liquid formulation disclosed herein is preferably an injection.

A "pharmaceutically acceptable carrier" refers to an ingredient in a pharmaceutical formulation, other than the active ingredient, which is not toxic to the subject. Pharmaceutically acceptable carriers include, but are not limited to, buffers, excipients, stabilizers, or preservatives.

As used herein, "buffer" refers to a pH buffer. For example, the buffer is selected from histidine, glutamate, phosphate, acetate, citrate, and tris(hydroxymethyl)aminomethane.

As used herein, the term "vehicle" refers to a liquid used to dissolve or suspend an active ingredient and an inactive ingredient to form a liquid formulation. Vehicles that can be used in the present invention include, but are not limited to, water for injection, organic solvents for injection including, but not limited to, oil for injection, ethanol, propylene glycol, and the like, or combinations thereof.

"sPGA" refers to static Physician's Global Assessment, which records the physician's assessment of the subject's psoriasis status, including the following aspects: induration, scaling, and erythema. It is used to evaluate the psoriasis skin lesions of a subject at a given time point. All skin lesions were graded according to the following ranges of induration, erythema, and scaling. The sPGA score is obtained by dividing the total score of the 3 items by 3, as shown below.

Induration (I) (average of all skin lesions; measured using the NPF National Psoriasis Foundation Reference card)

0 = No evidence of plaque elevation
1 = microplaque elevation, 0.25mm
2 = slight plaque elevation, 0.5 mm
3 = Moderate plaque elevation, 0.75 mm
4 = Marked plaque elevation, 1 mm
5 = Severe plaque elevation, $\geq$ 1.25 mm

Erythema (E) (average of all skin lesions)

0 = No evidence of erythema, hyperpigmentation may be present
1 = faint erythema
2 = slight red color
3 = Moderately red
4 = Bright Red
5 = Dark red to deep red

Scaling (S) (average of all skin lesions)

0 = No evidence of scaling
1 = Slight scaling; occasional scaling <5% of total skin lesions
2 = Mild; predominately fine scaling
3 = moderate; coarse scale predominates
4 = Marked; thick, non-recalcitrant scale predominates
5 = Severe; very thick and recalcitrant scales predominate

sPGA Overall Mean Score = I + E + S/3
sPGA (static Physician Global Assessment) is evaluated based on the total average score:

0 = clear, except for some residual discoloration
1 = minimal, most skin lesions are individually scored 1

2 = Mild, most skin lesions are individually scored 2

3 = moderate, most skin lesions are individually scored 3

4 = marked, most skin lesions are individually scored 4

5 = Severe, most skin lesions are individually scored 5

Note: The fraction should be rounded to the nearest whole number. If the total score is < 1.50, the score = 1; if the total score is ≥ 1.50, the score = 2.

sPGA-G uses the evaluation criteria of sPGA to conduct a local assessment of perineal psoriasis, with a score of 0-5.

"PASI" refers to Psoriasis Area and Severity Index, and is a systematic tool for assessing and grading the severity of psoriatic skin lesions and response to treatment. PASI scores range from 0 to 72. The percentage of body surface area affected can be linearly combined with the severity of erythema, induration, and scaling at the four body sites. This endpoint is based on percent reduction relative to the baseline, usually summarized as a binomial distribution based on achieving X% reduction (or PSAIx), where X can be 50, 75, 90, and 100. To calculate PASI, the areas of four major body parts were evaluated: head (h), trunk (t), upper extremities (u), and lower extremities (l), which account for 10%, 30%, 20% and 40% of the total body surface area respectively.

The areas affected by psoriasis in these four parts are expressed numerically:

0 = not involved;

1 = < 10%;

2 = 10% to < 30%;

3 = 30% to < 50%;

4 = 50% to < 70%;

5 = 70% to < 90%;

6=90%-100%

The erythema (E), induration (I), and scaling (S) of the skin lesion indicating the severity were evaluated using a scale of 0-4, where 0 represents no skin involvement at all, 1 is mild, 2 is moderate, 3 is severe, and 4 is very severe; the scores of the four sites were listed separately.

To aid area evaluation, note the following points:

a. Neck belongs to the head

b. Armpits and groins belong to trunk

c. Buttocks belong to the lower limbs

$$PASI = 0.1 * (E_h + I_h + S_h) A_h + 0.3 * (E_t + I_t + S_t) A_t + 0.2 * (E_u + I_u + S_u) A_u + 0.4 * (E_l + I_l + S_l) A_l.$$

"DLQI" refers to Dermatology Life Quality Index, is a dermatology-related quality-of-life instrument used to assess the impact of disease on the quality of life of subjects (AY Finlay and GK Khan). A questionnaire consisting of 10 questions is used to assess six different aspects of quality of life: symptoms and feelings, daily activities, leisure activities, work or school standards, personal relationships and treatment. The DLQI is a one-week recall period. Response categories included not problem (0), not at all (0), few (1), serious (2), very serious (3). The answer to question 7 was "Yes"/"No", "yes" is scored 3 points.

The DLQI is to be filled out by the subject independently during the visits marked in the flow chart.

The DLQI was analyzed by the following 6 aspects:

| Dimension | Issue No. | Score |
|---|---|---|
| Symptoms and Feelings | Questions 1 and 2 | Score up to 6 |
| Daily Activities | Questions 3 and 4 | Score up to 6 |
| Leisure | Questions 5 and 6 | Score up to 6 |
| Work and study | Q7 | Score up to 3 |
| Interpersonal relationships | Questions 8 and 9 | Score up to 6 |
| Treatment | Q10 | Score up to 3 |

Score corresponding to the option

| Very Much | Many | A little | Not at all | Not related | Not answered |
|---|---|---|---|---|---|
| 3 | 2 | 1 | 0 | 0 | 0 |

For item 7, select "Yes", if the score reaches the maximum of 3 points, select "No", and select the score in the table according to the options.

The DLQI total score was derived by summing each question score, with results ranging from 0 to 30. The higher the score, the worse the quality of life.

> 0-1 points: not affecting the subject's life,
> 2-5 points: small effect,
> 6-10 points: moderate impact,
> 11-20 points: very large impact,
> 21-30 points: having a great impact on the subject's life.

If a question answer is missing for a dimension, the dimension is considered missing. If 2 or more questions were not answered, the DLQI total score was considered missing. A change of 5 points from baseline was considered a clinically important difference.

As used herein, the term "single pharmaceutical dosage unit" refers to a single pharmaceutical dosage formulation comprising the antibody of the present invention to be administered to a subject at the time of administration, such as a vial, an ampoule, a pre-filled syringe or a pre-filled syringe for injection, which contains a solution or a lyophilized powder of the drug.

## Methods of treatment

The present invention provides a method for switching a subject who has previously received a biological agent for treating plaque psoriasis to an anti-IL23p19 antibody for treating plaque psoriasis.

In some specific embodiments of the present invention, the amino acid sequence of the heavy chain CDR1 of the anti-IL23p19 antibody comprises SEQ ID NO.1, the amino acid sequence of the heavy chain CDR2 comprises SEQ ID NO.2, and the amino acid sequence of the heavy chain CDR3 comprises SEQ ID NO.3; the amino acid sequence of the light chain CDR1 of the antibody comprises SEQ ID NO.4, the amino acid sequence of the light chain CDR2 comprises SEQ ID NO.5, and the amino acid sequence of the light chain CDR3 comprises SEQ ID NO.6.

In one specific embodiment of the present invention, the method comprises that the anti-IL-23p19 antibody is an antibody specifically binding to IL-23p19 and comprises the following 6 CDRs:

- heavy chain VH CDR1, as shown in GYTFTSYLMH (SEQ ID NO: 1);
- heavy chain VH CDR2, as shown in YINPYNEGTN (SEQ ID NO: 2);
- heavy chain VH CDR3, as shown in NWDLPY (SEQ ID NO: 3);
- light chain VL CDR1, as shown in RASQSISDYLH (SEQ ID NO: 4);
- light chain VL CDR2, as shown in YASQSMS (SEQ ID NO: 5); and
- light chain VL CDR3, as shown in QQGHSFPFT (SEQ ID NO: 6).

In one specific embodiment, the anti-IL-23p19 antibody comprises a heavy chain variable region VH and a light chain variable region VL, wherein the heavy chain variable region comprises the sequence of SEQ ID NO: 7 or a sequence having at least 90%, 95%, 98% or 99% identity thereto, and the light chain variable region comprises the sequence of SEQ ID NO: 8 or a sequence having at least 90%, 95%, 98% or 99% identity thereto:

Sequence (SEQ ID NO: 7)

QVQLVQSGAEVKKPGASVKVSCKASGYTFTSYLMHWVRQAPGQGLEW
MGYINPYNEGTNYAQKFQGRVTMTRDTSISTAYMELSRLRSDDTAVYYCAR
NWDLPYWGQGTLVTVSS ;

Sequence (SEQ ID NO: 8)

DIQMTQSPSSLSASVGDRVTITCRASQSISDYLHWYQQKPGKAPKLLIKY
ASQSMSGVPSRFSGSGSGSDFTLTISSLQPEDFATYYCQQGHSFPFTFGQGTKL
EIK。

In a specific embodiment, the IL-23p19 antibody is an IgG1 antibody comprising a heavy chain and a light chain, wherein the heavy chain comprises a sequence of SEQ ID NO: 9 or a sequence having at least 90%, 95%, 98% or 99% identity thereto, and the light chain comprises a sequence of SEQ ID NO: 10 or a sequence having at least 90%, 95%, 98% or 99% identity thereto.

Sequence (SEQ ID NO: 9)

QVQLVQSGAEVKKPGASVKVSCKASGYTFTSYLMHWVRQAPGQGLEW
MGYINPYNEGTNYAQKFQGRVTMTRDTSISTAYMELSRLRSDDTAVYYCAR
NWDLPYWGQGTLVTVSSASTKGPSVFPLAPSSKSTSGGTAALGCLVKDYFPEP
VTVSWNSGALTSGVHTFPAVLQSSGLYSLSSVVTVPSSSLGTQTYICNVNHKP
SNTKVDKKVEPKSCDKTHTCPPCPAPELLGGPSVFLFPPKPKDTLYITREPEVT
CVVVDVSHEDPEVKFNWYVDGVEVHNAKTKPREEQYNSTYRVVSVLTVLHQ
DWLNGKEYKCKVSNKALPAPIEKTISKAKGQPREPQVYTLPPSRDELTKNQVS
LTCLVKGFYPSDIAVEWESNGQPENNYKTTPPVLDSDGSFFLYSKLTVDKSRW
QQGNVFSCSVMHEALHNHYTQKSLSLSPG

Sequence (SEQ ID NO: 10)

DIQMTQSPSSLSASVGDRVTITCRASQSISDYLHWYQQKPGKAPKLLIKY
ASQSMSGVPSRFSGSGSGSDFTLTISSLQPEDFATYYCQQGHSFPFTFGQGTKL
EIKRTVAAPSVFIFPPSDEQLKSGTASVVCLLNNFYPREAKVQWKVDNALQSG
NSQESVTEQDSKDSTYSLSSTLTLSKADYEKHKVYACEVTHQGLSSPVTKSFN
RGEC

Preferably, the IL-23p19 antibody is the anti-IL-23p19 antibody 17D1-YTE disclosed in PCT Application No. PCT/CN2019/121261 (International Application Date: Nov. 27, 2019), which consists of a heavy chain sequence of SEQ ID NO: 9 and a light chain sequence of SEQ ID NO: 10.

In some specific embodiments, the anti-IL-23p19 antibody is recombinantly expressed in HEK 293 cells or CHO cells.

In some specific embodiments, the formulation of the IL - 23p19 antibody can be prepared according to the formulation containing an anti - IL23p19 antibody, its preparation method, and uses disclosed in PCT Application No. PCT/CN2021/093219 (International Filing Date: May 12, 2021).

In some specific embodiments of the present invention, the method comprises administering to a subject previously treated with a biological agent for plaque psoriasis an anti-IL-23p19 antibody, wherein the anti-IL-23p19 antibody is administered at weeks 0, 4, and 8, respectively, and once every 12 weeks thereafter to a subject who has lost response or has an poor response to biological agent.

Wherein, the dosage for each administration is 100 mg to 1000 mg. For example, the dosage for each administration is 100 mg, 200 mg, 300 mg, 400 mg, 500 mg, 600 mg, 700 mg, 800 mg, 900 mg, or 1000 mg. Preferably, the dosage for each administration is 200 mg.

Among them, poor response was defined as: sPGA $\geq$ 2 points, or cumulative involved body surface area of skin lesions $\geq$ 3%, or failure to achieve 75% improvement in PASI after the use of biological agents.

In some specific embodiments of the present invention, the method comprises administering an anti-IL-23p19 antibody to a subject who has previously been treated with a biological agent for plaque psoriasis, and administering the anti-IL-23p19 antibody at week 0 and every 12 weeks thereafter to a subject who responds to the biological treatment. wherein the dosage for each administration is 100 mg to 1000 mg. Specifically, the dosage for each administration is 100 mg, 200 mg, 300 mg, 400 mg, 500 mg, 600 mg, 700 mg, 800 mg, 900 mg, or 1000 mg. Preferably, the dosage for each administration is 200 mg.

Response is defined as: an sPGA score of 0 or 1, and cumulative involved body surface area of skin lesions of < 3%; or a PASI improvement of 75% or greater after the use of biological agents.

In some specific embodiments of the present invention, the route of administration is subcutaneous injection.

In some specific embodiments of the present invention, the biological agent comprises a tumor necrosis factor $\alpha$ inhibitor/antagonist drug and/or an IL-17A inhibitor/antagonist drug. The TNF-$\alpha$ inhibitor/antagonist drugs include etanercept, infliximab, adalimumab, golimumab, and certolizumab. The IL-17A inhibitor/antagonist drugs include secukinumab and ixekizumab.

Still further, the TNF-$\alpha$ inhibitor/antagonist drugs include, but are not limited to, etanercept, infliximab, and adalimumab. The IL-17A inhibitor/antagonist drugs include, but are not limited to, secukinumab.

For example, previous treatment with biological agents:

a. Those who receive regular treatment according to the prescribed dosage should meet:

-    Use of IL-17 inhibitors for at least 4 months: subcutaneous injection of secukinumab at weeks 0, 1, 2, 3 and 4, and every 4 weeks thereafter at a dose of 300 mg; subcutaneous injection of ixekizumab at week 0 at a dose of 160 mg, and at weeks 2, 4, 6, 8, 10 and 12, and every 4 weeks thereafter at a dose of 80 mg;
-    Use of TNF inhibitors for at least 4 months: adalimumab 80 mg subcutaneously at week 0, 40 mg subcutaneously at week 1 and every 2 weeks thereafter; etanercept 25 mg twice weekly for the first 3 months, then 50 mg once weekly or 25 mg twice weekly after 3 months; infliximab 5 mg/kg intravenously at weeks 0, 2, and 6, and the same dose every 8 weeks thereafter;

or

b. Those who do not receive regular treatment according to the prescribed dosage, refer to those who do not meet the dosing amount and dosing interval requirements in the previous item, but the time from the last administration of the biological agent to the first administration of the biological agent is at least 4 months.

**Single Pharmaceutical Dosage Unit**

A single pharmaceutical dosage unit, comprising: an effective amount of the anti-IL-23p19 antibody of the present invention, preferably comprising a fixed dose selected from 100 mg-1000 mg; preferably 50 mg-800 mg, more preferably a fixed dose of 50 mg-500 mg, e.g., 50 mg, 60 mg, 70 mg, 80 mg, 90 mg, 100 mg, 110 mg, 120 mg, 130 mg, 140 mg, 150 mg, 160 mg, 170 mg, 180 mg, 190 mg, 200 mg, 210 mg, 220 mg, 230 mg, 240 mg, 250 mg, 260 mg, 270 mg, 280 mg, 290 mg, 300 mg, 350 mg, 400 mg, 450 mg, 500 mg, 550 mg, 600 mg, 650 mg, 700 mg, 750 mg, 800 mg, 850 mg, 900 mg, 950 mg, or 1000 mg, preferably 50 mg, 60 mg, 70 mg, 80 mg, 90 mg, 100 mg, 150 mg, 200 mg, 300 mg, 350 mg, 400 mg, or 500 mg of the anti-IL-23p19 antibody.

In some embodiments, the single pharmaceutical dosage unit is a single pharmaceutical dosage unit packaged in a prefilled automated injection pen.

Pharmaceutical kit

A pharmaceutical kit comprising an effective amount of the anti-IL-23p19 antibody disclosed herein, preferably comprising a fixed dose of 100-1000 mg, and more preferably comprising 100 mg, 200 mg, 300 mg, 400 mg, 500 mg, 600 mg, 700 mg, 800 mg, 900 mg or 1000 mg of the anti-IL-23p19 antibody. Specifically, it also comprises a package insert printed with instructions on the use of the anti-IL-23p19 antibody for the prevention or treatment of plaque psoriasis in subjects.

In some embodiments, the pharmaceutical kit comprises the anti-IL-23p19 antibody in a single pharmaceutical dosage unit form. In other embodiments, the single pharmaceutical dosage unit is packaged in a prefilled automatic injection pen.

**Applications**

Use of the anti-IL-23p19 antibody, the single pharmaceutical dosage unit, or the pharmaceutical kit disclosed herein in the manufacture of a medicament for preventing or treating plaque psoriasis.

## EXAMPLES

### Example 1. Preparation and purification of IL-23p19 antibody

The antibody 17D1-YTE specifically binding to IL-23p19 was obtained as described in PCT Application No. PCT/CN2019/121261. The antibody has a heavy chain sequence of SEQ ID NO: 9 and a light chain sequence of SEQ ID NO: 10. PCT Application No. PCT/CN2019/121261 is incorporated herein by reference in its entirety.

Briefly, the antibody is recombinantly expressed in CHO cells. The IL-23p19 antibody samples used in the present invention are purified by affinity chromatography, and the IL-23p19 antibody samples used in the formulation screening experiments of the present invention are purified by cation exchange chromatography.

### Example 2. Clinical Studies

The results of preclinical pharmacokinetics (PK), pharmacodynamics (PD), and toxicology studies showed that the anti-IL-23P19 antibody had a clear target, a clear mechanism of action, and significant anti-inflammatory effects, and was expected to reduce or clear the skin lesions of subjects with plaque psoriasis, improve the severity of the skin lesions of the subjects, and reduce the cumulative body surface area of the skin lesions. The anti-IL-23P19 antibody exhibited linear PK characteristics in cynomolgus monkeys, with a long half-life, high bioavailability, high in vivo safety after short-term or long-term repeated administration in cynomolgus monkeys, and no risk of hemolysis or cytokine release in vitro.

The randomized, double-blind, placebo-controlled, phase I clinical study on the safety and tolerability of single-dose escalation in healthy subjects has been completed for the anti-IL-23p19 antibody of the present invention. The results show that the anti-IL-23P19 antibodies were safe and well tolerated in healthy humans.

The anti-IL-23p19 antibody of the present invention has completed a dose-ranging phase II multicenter, randomized, double-blind, placebo-controlled clinical study in moderate to severe plaque psoriasis and has reached the primary efficacy endpoint. The results of the phase II study showed that the anti-IL-23p19 monoclonal antibody could significantly improve the skin lesions of subjects with moderate to severe plaque psoriasis and had good overall safety and tolerability.

The results of the above two studies verified the significant efficacy and safety of the anti-IL-23P19 antibody in Chinese subjects with moderate to severe plaque psoriasis, and the benefits outweigh the risks. The data support the continued clinical study of this product.

This study is a multi-center, open-label study of anti-IL-23p19 monoclonal antibody to evaluate the efficacy of switching subjects with plaque psoriasis previously treated with biological agents to the anti-IL-23p19 monoclonal antibody of the present invention.

3.1 Study Objectives

• Primary Objective:

To evaluate the efficacy of switching subjects previously treated with biological agents for plaque psoriasis to the anti-IL23p19 antibody treatment of the present invention.

• Secondary objective

- To evaluate the safety of switching to anti-IL-23P19 antibody therapy in subjects with plaque psoriasis treated with biological agents;
- To evaluate the impact of switching to anti-IL-23P19 antibody therapy on health-related quality of life in subjects with plaque psoriasis treated with biological agents;
- To evaluate the immunogenicity of switching to anti-IL-23P19 antibody therapy in subjects with plaque psoriasis treated with biological agents.

3.2 Study Endpoints

• Primary efficacy endpoint

. The proportion of subjects who achieve a Static Physician's Global Assessment (sPGA) score of clear (0 points) or almost clear (1 point) and have a BSA< 3% at Week 16.

• Secondary efficacy endpoints

- The proportion of subjects who achieve a Static Physician's Global Assessment (sPGA) score of clear (0 points) at Week 16;
- The proportion of subjects who achieve a Dermatology Life Quality Index (DLQI) score of 0/1 at Week 16;
- The proportion of subjects with an sPGA score of 0 or 1, an sPGA score of 0, and a DLQI score of 0 or 1 at Week 44;
- The proportion of subjects with an sPGA score of 0 or 1, an sPGA score of 0, and a DLQI score of 0 or 1 at Week 16 who maintain the relevant scores at Week 44.

• Other efficacy endpoints

- Change in DLQI from baseline;
- Change in Psoriasis Area and Severity Index (PASI) from baseline;
- Change in cumulative BSA of skin lesions from baseline;
- Proportion of subjects with BSA $\leq$ 1% and $\leq$ 3%;
- Proportion of subjects with PASI $\leq$1 and $\leq$3 (only for those with poor responses at baseline);
- Time to sPGA 0 or 1 (only for those with poor response at baseline);
- Time to sPGA 0 (only for those with poor response at baseline);

• Safety endpoints

All adverse events, serious adverse events, etc. Changes in the results of vital signs, physical examinations, laboratory tests, electrocardiograms, etc. before and after drug administration.

• Immunogenicity evaluation

The development of anti - drug antibodies (ADA) and neutralizing antibodies (NAb).

3.3 Study Design

This is a multi-center, open-label study mainly evaluating the efficacy of switching subjects treated with biological agents for plaque psoriasis to the anti-IL23p19 antibody of the present invention.

The target population is male or female subjects with plaque psoriasis aged between 18 and 75 years old, who have previously used biological agents for the treatment of plaque psoriasis before screening, including IL - 17 inhibitors and tumor necrosis factor - $\alpha$ (TNF - $\alpha$) inhibitors. Before screening, subjects can either be those who are regularly treated according to the prescribed dose (for at least 4 months) or those who are not treated regularly according to the prescribed dose (with at least 4 months between the last and the first administration of biological agents).

It is planned to enroll approximately 160 subjects, and the proportion of subjects with poor response at baseline should be no less than 30% of the total enrolled subjects. Subjects with poor response at baseline (sPGA $\geq$ 2 points, or cumulative body surface area of skin lesions $\geq$ 3%, or PASI improvement less than 75% after using biological agents) will be switched to the anti-IL23p19 antibody of the present invention. They will receive 200 mg of the anti-IL23p19 antibody by subcutaneous injection at Weeks 0, 4, and 8, and then once every 12 weeks until the last administration at Week 32. Responders (sPGA of 0 or 1 point, and cumulative body surface area of skin lesions < 3%, or PASI improvement of 75% or more after using biological agents) will also be switched to the anti - IL23p19 antibody of the present invention. They will receive the first dose at Week 0 and then once every 12 weeks until the last administration at Week 36. The time to start switching to the anti - IL23p19 antibody treatment should be within 1 week before or after the scheduled administration date of the biological agents. The screening period of the study shall not exceed 4 weeks. Eligible subjects will be switched to the anti - IL23p19 antibody treatment of the present invention, and follow- up will end at Week 44 after the last administration.

3.4 Inclusion Criteria

Eligible subjects must meet all of the following inclusion criteria:

(1) Male or female aged 18 to 75 years;

(2) The investigator assessed that the subject was suitable to continue the treatment of plaque psoriasis with biological agents;

(3) Previous treatment with biological agents

    a. Those who are regularly treated according to the prescribed dose should meet:

- Use of IL-17 inhibitors for at least 4 months: subcutaneous injection of 300 mg secukinumab at weeks 0, 1, 2, 3, and 4, and every 4 weeks thereafter; subcutaneous injection of ixekizumab at week 0 at 160 mg, and at weeks 2, 4, 6, 8, 10, and 12, and every 4 weeks thereafter at 80 mg;
- Use of TNF inhibitors for at least 4 months: subcutaneous injection of 80 mg adalimumab at week 0, 40 mg at week 1 and every 2 weeks thereafter; 25 mg etanercept twice weekly for 3 months, then 50 mg once weekly or 25 mg twice weekly for 3 months; 5 mg/kg infliximab intravenously at weeks 0, 2, and 6, then the same dose every 8 weeks;

    or

b. Those who are not treated regularly according to the prescribed dose, refer to those who do not meet the previous dose and interval of administration, but the last dose of biological agents is at least 4 months from the first dose of biological agents;

(4) Those who respond or poorly respond to biological therapy at screening and baseline:

    a. Response is defined as: sPGA of 0 or 1 and cumulative BSA of skin lesion of < 3%; or PASI improvement of 75% or greater after the use of biological agents;
    b. Poor response is defined as: sPGA $\geq$ 2 points, or cumulative BSA of skin lesion $\geq$ 3%, or failure to achieve 75% improvement in PASI after the use of biological agents;

(5) Fully understand the purpose of the trial, have a basic understanding of the pharmacological effects of the test drug and possible adverse reactions; voluntarily sign the informed consent in accordance with the spirit of the Declaration of Helsinki.

3.5 Exclusion Criteria

Eligible subjects did not meet any of the following exclusion criteria:

(1) Previous or current diagnosis of pustular psoriasis, erythrodermic psoriasis, or drug-induced psoriasis (e.g., psoriasis caused by beta blockers, calcium channel inhibitors, etc.); or guttate psoriasis at screening or before the first dose;
(2) Those who have previously used anti-IL23p19 antibodies and IL-23 preparations;
(3) Those who have received two biological agents for the treatment of psoriasis within 4 months before screening;
(4) Those who have used local treatment for psoriasis within 2 weeks before the first administration, or those who have used non-biological systemic treatment or phototherapy within 4 weeks before the first administration;
(5) Use of Natalizumab, or B-cell or T-cell modulators (e.g., Rituximab, Abatacept, or Visilizumab) within 12 months prior to the first dose;
(6) Unwillingness to avoid continuous sun exposure and other ultraviolet light sources during the study period;
(7) Received treatment with an experimental biological agent within 6 months prior to the first dose of the study drug, or received any experimental treatment within 30 days, or received the study drug within 5 half-lives, or is participating in a clinical study;
(8) There is evidence that the subject has severe, progressive, uncontrolled (including but not limited to) cardiovascular disease, neuromuscular disease, blood disease, respiratory disease, liver or digestive disease, urinary disease, neurological or mental disease;
(9) opportunistic infections [e.g., herpes zoster (severe or recurrent), active cytomegalovirus, pneumocystis carinii, histoplasma, aspergillus, mycobacteria, etc.] within 6 months prior to screening;
(10) Known history of recurrent or chronic infection, history of chronic or recurrent infection, including but not limited to: chronic kidney infection, chronic chest infection (such as bronchiectasis), recurrent urinary tract infection, open, drainage or skin infectious wounds;
(11) History of serious infection (e.g., sepsis, pneumonia, pyelonephritis), or hospitalization for infection within 2 months prior to screening;
(12) A history of malignant tumor or malignancy (except for squamous cell carcinoma of the skin, basal cell carcinoma, or localized cervical carcinoma in situ that has been successfully resected and has no evidence of recurrence or metastasis within 5 years);

(13) Suffering from or have suffered from lymphoproliferative disease, or have symptoms or signs suggestive of lymphoproliferative disease within 5 years before screening, such as lymph nodes and/or splenomegaly;

(14) Known history of active tuberculosis, or suspected tuberculosis based on clinical manifestations or imaging evidence of tuberculosis (including but not limited to pulmonary tuberculosis, lymphoid tuberculosis, tuberculous pleurisy, etc.), or tuberculosis status of subjects undergoing interferon gamma release assay (IGRA) during the screening period. For subjects without symptoms of active tuberculosis and imaging evidence of tuberculosis:

- If the IGRA test result is negative, the subject meets eligibility;
- If the IGRA test result is indeterminate, a retest can be performed. If the retest result is still indeterminate, the subject does not meet the eligibility criteria;
- If the IGRA test result is positive, the subject may be screened and evaluated again after receiving prophylactic anti-tuberculosis treatment for not less than 1 month. If the patient has no symptoms of tuberculosis and is well tolerated to tuberculosis drugs, and is willing to receive complete prophylactic anti-tuberculosis treatment during the study period, the patient may be enrolled as assessed by the investigator;
- Subjects with a positive IGRA result prior to biologic therapy who have received complete prophylactic antituberculous therapy and have no symptoms of active tuberculosis or evidence of radiographic tuberculosis are eligible for enrollment.

(15) Have received Bacillus Calmette - Guérin (BCG) vaccination within 12 months before the first use of the study drug, or plan to receive BCG vaccination during the study period or within 12 months after the last study treatment.;

(16) Receive live or bacterial vaccines within 3 months prior to the first dose of the study drug, or plan to receive live or bacterial vaccines during the study period or within 3 months after the last study treatment;

(17) Hematology and blood biochemistry results during the screening period and baseline period meet the following conditions:

- Any parameter of hemoglobin, red blood cells, white blood cells, neutrophils, and platelets < lower limit of normal value (LLN), and the abnormality is judged by the investigator to be of clinical significance.;
- Any parameter of Alanine Aminotransferase (ALT), Aspartate Amino Transferase (AST), Total Bilirubin (TBIL), or Direct Bilirubin (DBIL) > 2 times the upper limit of normal value (ULN);
- Creatinine (Cr) > ULN; patients who meet the protocol requirements after reexamination may also be enrolled.

(18) The results of virus testing at the time of screening meet any of the following criteria:

- Human immunodeficiency virus (HIV) antibody positive;
- Hepatitis C virus (HCV) antibody positive with no history of successful treatment, with successful treatment defined as HCV RNA negative after at least 24 weeks of antiviral treatment;
- Hepatitis B virus (HBV) screening includes at least hepatitis B surface antigens (HBsAg), hepatitis B surface antibodies (HBsAb), and hepatitis B core antibodies (HBcAb), and if HBsAg is positive; or if only HBcAb is positive when HBsAg is negative, HBV DNA should be tested and the result is positive;
- Positive for syphilis specific antibody (except for those whose syphilis nonspecific antibody titer turns negative after regular syphilis treatment).

(19) Clinically significant 12-lead electrocardiogram (ECG) abnormalities at screening: QTcF > 450 ms, shortened or delayed PR interval, second or third degree atrioventricular block, pre-excitation syndrome, long QT syndrome; or severe arrhythmia requiring treatment;

(20) Previous severe drug or food allergy, and/or allergy to the test drug or its components;

(21) History of alcohol and drug abuse within 12 months prior to screening;

(22) Female subjects who are pregnant or breastfeeding, or women of gestational age who have a positive pregnancy test at screening and before administration;

(23) Those who plan to have children during the study and within 6 months after the study drug administration, or are unwilling to take appropriate contraceptive measures (such as condoms) as deemed by the doctor during the study;

(24) Those whom the researcher deems unsuitable to participate in this clinical trial due to various reasons..

3.6 Study Drug, Dose, and Mode of Administration

Poor responders: subcutaneous injection of 200 mg of the anti-IL23p19 antibody of the present invention at weeks 0, 4, 8, 20, and 32;

Responders: 200 mg of the anti-IL23p19 antibody of the present invention was injected subcutaneously at weeks 0, 12, 24 and 36.

Dosage form of the study drug: injection. The specification is 100 mg (1 mL)/vial. The formulation is as follows: 100.0 mg/mL recombinant anti-interleukin 23p19 subunit antibody, 0.76 mg/mL histidine, 1.08 mg/mL histidine hydrochloride, 50.00 mg/mL sorbitol, 0.5 mg/mL polysorbate 80, pH 6.0.

The following treatments were prohibited during the treatment period:

• Drugs and treatments prohibited before the first dose follow the relevant requirements in the exclusion criteria, and are still prohibited during the study. Prohibited medications/therapies during the study are listed in the table below:

Table: Prohibited Medications/Therapies During the Study

| |
|---|
| • TNF-$\alpha$ antagonists |
| • IL-17 |
| • Drugs targeting IL-23 (except anti-IL-23P19 antibodies) |
| • Natalizumab, or B-cell or T-cell modulating agents (e.g., Rituximab, Abatacept, or Visilizumab) |
| • Other systemic therapeutic drugs for psoriasis (including but not limited to methotrexate, cyclosporine, retinoids, azathioprine, leflunomide, mycophenolate mofetil, sulfasalazine, glucocorticoids, JAK inhibitors such as tofacitinib, baricitinib, or traditional Chinese medicine or Chinese patent medicine for psoriasis) |
| • Topical therapeutic drugs for psoriasis (including but not limited to glucocorticoids, vitamin D3 derivatives, retinoids, calcineurin inhibitors, keratolytic agents, and compound preparations) |
| • Phototherapy for psoriasis |
| • Experimental biological agents |
| • Other experimental drugs |

Treatment should be discontinued if the subject uses a prohibited medication that has been assessed to have a significant impact on efficacy and safety (e.g., use of biological agents, potent or super-potent local therapy for more than 1 week, or non-biologic systemic drugs).

During administration, drugs with hepatotoxicity should be avoided.

3.7 Treatment Compliance

The subjects received the study treatment at the study site, and their treatment compliance was monitored using drug dispensation records, subject medical records, and eCRFs.

3.8 Indicators related to efficacy evaluation

A. Psoriasis Area and Severity Index (PASI)

As far as possible, ensure that the doctor who performs the PASI assessment on the subject at the screening/baseline stage conducts the PASI assessment on this subject during subsequent follow-up visits.

PASI 90 is defined as a $\geq$ 90% improvement in PASI score from baseline; PASI 75 is defined as a $\geq$ 75% improvement in PASI score from baseline; PASI 100 is defined as a 100% improvement in PASI score from baseline.

B. Static Physician Global Assessment (sPGA)

sPGA records the physician's assessment of the subject's psoriasis status, including the following aspects: induration, scaling, and erythema.

Whenever possible, the physician performing the sPGA assessments at Screening/Baseline performed the sPGA assessments for the subject at subsequent visits.

C. Dermatology Life Quality Index (DLQI)

For visits involving the DLQI assessment, the DLQI assessment was performed before all other visit contents (examination, procedures, psoriasis assessment, collection of adverse events and concomitant medications, etc.) during the study.

D. Skin lesion photography

In order to obtain more efficacy evaluation data, the subject's skin condition (except for the perineum) will be photographed each time when the efficacy evaluation is performed.

3.9 Safety Assessment

I. Laboratory Tests

Routine laboratory safety assessments include the laboratory tests listed in the table below.

**Contents of routine laboratory tests**

| | |
|---|---|
| Hematology | Red blood cell (RBC) count, hemoglobin (HGB), white blood cell (WBC) count, platelet (PLT) count, neutrophil percentage, lymphocyte percentage, monocyte percentage. |
| Blood biochemistry | Aspartate Aminotransferase (AST), Alanine Aminotransferase (ALT), Total Bilirubin (TBIL), Direct Bilirubin (DBIL), Total Protein (TP), Albumin (ALB), Fasting Blood Glucose (FBG), Alkaline Phosphatase, Serum Potassium, Serum Sodium, Serum Calcium, Serum Phosphorus, Uric Acid, Triglyceride (TG), Lactate Dehydrogenase (LDH), Creatine Kinase (CK), Urea (UREA)/Blood Urea Nitrogen (BUN), And Creatinine (Cr). |
| Urinalysis | Urine Protein (UPRO), Urine Glucose (UGLU), Urinary Red Blood Cells (URBC), Urinary White Blood Cells (UWBC), Ketone (KET), Specific Gravity (SG), Uric Alkalinity (Ph), Urobilinogen (URO), and Urinary Nitrite (NIT). |

| | Hepatitis B panel: HBsAg, HBsAb, HBcAb, HBeAb, and HBeAg; HCV antibody and HIV antibody. If necessary, HBV-DNA, HCV-RNA; syphilis-specific antibodies and non-specific antibodies may be tested. Eligibility requirements for hepatitis B test indicators: | | | |
|---|---|---|---|---|
| Viral serology | HBsAg | HBsAb | HBcAb | ELIGIBILITY |
| | + | + (or -) | + (or -) | Not compliant |
| | - | + | + (or -) | Complies |
| | - | - | + | Detect HBV-DNA; the result below the lower limit of quantitation shall comply with the requirements |
| | - | - | - | Complies |
| Tuberculosis test | Interferon gamma release assays (IGRAs) include QuantiFERON-TB or T-SPOT. | | | |
| Pregnancy test | Blood pregnancy test or urine pregnancy test. | | | |

HBcAb, hepatitis B core antibody; HBeAb, hepatitis B e antibody; HBeAg, hepatitis B e antigen; HBsAb, hepatitis B surface antibody; HBsAg, hepatitis B surface antigen; HBV, hepatitis B virus; HCV, hepatitis C virus; HIV, human immunodeficiency virus.

Relevant laboratory tests will be performed under fasting conditions.

II. Clinical Examination

Clinical examinations include general physical examination, vital signs (including temperature, pulse, and blood pressure), 12-lead electrocardiogram (12-lead ECG), chest radiography, serious allergic reactions, injection site reactions, cardiovascular events (including major cardiovascular events (e.g., cardiovascular- and cerebrovascular-related death, non-fatal myocardial infarction, and non-fatal stroke) and other cardiovascular events known in the art), and psychiatric-related events.

3.10 Immunogenicity

About 5 mL of whole blood was collected, and the serum was separated, aliquoted, and cryopreserved for ADA and NAb analysis.

3.11 Statistical Analysis Methods

A. General Methods of Statistical Analysis

In this trial, the efficacy and safety data will be summarized separately according to whether the subjects have responded to previous biological agents..

No inter-group comparison was performed in this study. Measurement data will be described using number of subjects, mean, standard deviation, median, Q1, Q3, minimum, and maximum; count data will be described using frequency and percentage.

All statistical analyses were performed using SAS v9.4 (or higher).

B. Subject Baseline Characteristics

Describe and statistically analyze the demographic or baseline characteristics of the subjects, as well as the diagnosis and treatment information of the studied disease, medical history, previous concomitant treatments, etc..

C. Analysis of Efficacy

The evaluation of efficacy indicators will be summarized based on the safety analysis set according to the presence or absence of response.

1) Analysis of primary efficacy endpoint

The primary efficacy endpoint in this study was the proportion of subjects who achieved a sPGA score of 0 (clean) or 1 (near-clean) at Week 16. Subjects who do not have a sPGA score at Week 16 or have received other psoriasis treatments that affect the efficacy evaluation prior to Week 16 will be considered as sPGA non-target cases at Week 16.

The number and percentage of subjects achieving sPGA 0/1 was calculated and the 95% CI was calculated using the Clopper-Pearson method.

2) Analysis of secondary efficacy endpoints

Binary variable analysis

The secondary efficacy endpoints for binary variables include the proportions of subjects with a sPGA score of 0 and a DLQI score of 0/1 at Week 16; the proportions of subjects with a sPGA score of 0/1, a sPGA score of 0, and a DLQI score of 0/1 at Week 44; the proportions of subjects who maintained the relevant scores at Week 44 among those with a sPGA score of 0/1, a sPGA score of 0, and a DLQI score of 0/1 at Week 16; the proportions of subjects with a BSA $\leq$1% and $\leq$3%; the proportions of subjects with a PASI $\leq$ 1 and $\leq$ 3 (only for those with a poor response at baseline)..

The analysis method was the same as that for the primary efficacy endpoint.

Analysis of continuous variables

Secondary efficacy endpoints for continuous variables included changes from baseline in DLQI, PASI, and cumulative area of skin lesion (BSA) at each visit.

Summarize the descriptive statistics of each continuous efficacy endpoint by visit, including the number of cases, mean, standard deviation, median, Q1, Q3, minimum, and maximum. Also calculate the 95% confidence interval (CI) of the mean. Additionally, draw a graph showing the change of the mean over time.

Analysis of the time-to-event variable (only for subjects with a poor response at baseline)

The secondary efficacy endpoints of the time-to-event variable include the time to reach an sPGA score of 0/1 and an sPGA score of 0, which is defined as the time from the first administration of the study drug to the time when the sPGA score of 0/1 or 0 is achieved. If a subject fails to reach the corresponding sPGA score at the completion/termination of the study, the data will be censored at the time of the subject's last efficacy assessment.

Summarize the number and percentage of subjects who reach the corresponding sPGA scores and those with censored data. Use the Kaplan - Meier method to calculate the median time and its 95% CI, and draw a KM curve.

D. Safety Analysis

The safety analysis will be based on the safety analysis set.

1). Drug exposure

Summarize the subjects' exposure to the study drug, duration of drug use, compliance, etc. during the study period..

2). Adverse event

Adverse events were coded using the Medical Dictionary for Regulatory Activities (MedDRA). The number and percentage of subjects with various categories of AEs (including treatment-emergent adverse events, drug-related adverse events, AESIs, SAEs, etc.) will be summarized, and the occurrence of various AEs will be further summarized by MedDRA system organ class and preferred term. The relationship and severity of each AE with the investigational drug were also analyzed.

3). Laboratory Tests

For blood routine, blood biochemistry and other indicators, describe the measured values and the changes from the baseline at each time point using the number of cases, mean, standard deviation, median, Q1, Q3, minimum and maximum by visit. Use cross - classification tables to describe the changes between normal and abnormal status before and after drug administration.

For urinalysis, use cross - classification tables to describe the changes between normal and abnormal status before and after treatment..

4). 12-lead ECG

Summarize the measured values and the changes from the baseline of the quantitative ECG indicators using descriptive statistics. Use cross-classification tables to summarize the changes between normal and abnormal status before and after treatment.

5). Vital Signs, Physical Examination, and Other Safety-Related Tests

Vital sign measurements and changes from baseline will be summarized using descriptive statistics.

Subjects with abnormal changes from baseline in physical examination and other safety examinations will be listed.

E. Immunogenicity

The positive rates of anti-drug antibody (ADA) and neutralizing antibody (NAb) of anti-IL-23P19 antibodies throughout the study will be summarized using descriptive statistics.

3.12 Results

The above content demonstrates that the method of switching subjects with plaque psoriasis who have previously received biologic agent treatment to the anti - IL23p19 antibody treatment of the present invention exhibits excellent efficacy and safety.

- The anti-IL-23P19 antibody had good safety: In the single-dose study in healthy subjects and the multiple-dose study in psoriasis population, the overall safety of the subjects was good, the incidence of AEs was not significantly increased compared with that of the placebo group, and no dose-dependent safety events were observed.
- Increasing the loading dose exposure (average concentration) can increase the PASI 90 achievement rate at week 16: increasing the average concentration ($C_{avg, 16w}$) of the anti-IL-23P19 antibody within 16 weeks can increase the PASI 90 achievement rate in the psoriasis population at week 16. According to the simulation results of the robust PopPK/PASI model, about 57.5% of the psoriasis population achieved PASI 90 at week 16 after subcutaneous injection of 200 mg of anti-IL-23P19 antibody at weeks 0, 4, and 8.
- Long-interval (Q12W) maintenance dosing can maintain long-term benefits in the psoriasis population: According to the simulation results of the robust PopPK/PASI model, the maintenance dosing regimen of 200 mg Q12W was used, and the long-term (Week 52) PASI90 achievement rate was about 88%. The long-term maintenance dosing regimen is expected to provide the best convenient treatment regimen and stable efficacy treatment regimen for the psoriasis population.

The efficacy of switching subjects with plaque psoriasis who previously received interleukin-17 antagonists or anti-tumor necrosis factor antagonists to the anti-IL23p 19 antibody treatment of the present invention.

Subjects judged to be in clinical response at baseline received subcutaneous injection of 200 mg of anti-IL23p19 antibody once every 12 weeks; subjects judged to be in poor clinical response at baseline received subcutaneous injection of 200 mg of anti-IL23p19 antibody at weeks 0, 4, and 8, and every 12 weeks thereafter. The results are shown in the table below:

| | Week 4 | Week 8 | Week 12 | Week 16 | Week 20 | Week 24 | Week 28 | Week 32 | Week 36 |
|---|---|---|---|---|---|---|---|---|---|
| Clinical Responders % | 91.3% | 87.0% | 77.9% | **85.3%** | 86.6% | 83.6% | 86.0% | 84.3% | 85.3% |
| Clinical non | 10.8% | 26.5% | 45.6% | **52.1%** | 47.8% | 54.5% | 58.2% | 54.7% | 64.5% |

(continued)

|  | Week 4 | Week 8 | Week 12 | Week 16 | Week 20 | Week 24 | Week 28 | Week 32 | Week 36 |
|---|---|---|---|---|---|---|---|---|---|
| Responders % |  |  |  |  |  |  |  |  |  |

The study data show that after the subjects who were in clinical response at baseline were switched to the anti-IL23P19 antibody of the present invention, 85.3% of the subjects still maintained clinical response (the cumulative body surface area of skin lesions was < 3%, and the static physician global assessment score sPGA was 0 or 1) at week 16, and maintained at about 85% from week 16 to week 36 of observation; after the subjects who were not in clinical response at baseline were switched to the anti-IL23P19 antibody, the proportion of subjects who achieved response gradually increased, and the proportion of subjects who achieved response increased to 52.1% at week 16, after which a similar proportion of subjects still maintained response.

It can be seen that the anti-IL23p19 antibody of the present invention can effectively treat subjects who have previously received interleukin 17 antagonists or anti-tumor necrosis factor antagonists for the treatment of plaque psoriasis.

The exemplary embodiments of the present invention have been described above, and it should be understood by those skilled in the art that these disclosures are merely exemplary and that various other substitutions, adaptations, and modifications can be made within the scope of the present invention. Accordingly, the invention is not limited to the specific embodiments set forth herein.

## Claims

1. A method for treating plaque psoriasis in a subject, comprising administering to the subject an effective amount of an anti-IL23p19 antibody, wherein,
   the anti-IL23p19 antibody comprises a heavy chain variable region and a light chain variable region, wherein the amino acid sequence of CDR1 of the heavy chain variable region comprises SEQ ID NO: 1, the amino acid sequence of CDR2 of the heavy chain variable region comprises SEQ ID NO: 2, and the amino acid sequence of CDR3 of the heavy chain variable region comprises SEQ ID NO: 3; the amino acid sequence of CDR1 of the light chain variable region comprises SEQ ID NO: 4, the amino acid sequence of CDR2 of the light chain variable region comprises SEQ ID NO: 5, and the amino acid sequence of CDR3 of the light chain variable region comprises SEQ ID NO: 6.

2. The method according to claim 1, wherein the anti-IL23p19 antibody comprises a heavy chain variable region and a light chain variable region, wherein the heavy chain variable region comprises the sequence of SEQ ID NO: 7 or a sequence having at least 90%, 95%, 98% or 99% identity thereto, and the light chain variable region comprises the sequence of SEQ ID NO: 8 or a sequence having at least 90%, 95%, 98% or 99% identity thereto.

3. The method according to claim 1 or 2, wherein the anti-IL23p19 antibody comprises a heavy chain and a light chain, wherein the heavy chain comprises the sequence set forth in SEQ ID NO: 9 or a sequence having at least 90% identity to SEQ ID NO: 9, and the light chain comprises the sequence set forth in SEQ ID NO: 10 or a sequence having at least 90% identity to SEQ ID NO: 10.

4. The method according to any one of claims 1-3, wherein the subject has received biologic agent treatment for plaque psoriasis before the treatment.

5. The method according to any one of claims 1-4, wherein the subject has regularly received the biologic agent at the prescribed dosage (e.g., for at least 4 months)..

6. The method according to any one of claims 1-4, wherein the subject has not regularly received the biological agent at the prescribed dosage (e.g., at least 4 months from the last dose of the biological agent to the first dose of the biological agent).

7. The method according to any one of claims 1-6, wherein the subject has lost response or exhibited poor response to previous biological therapy.

8. The method according to claim 7, wherein the subject has an sPGA score of $\geq 2$, or a cumulative body surface area of skin lesions of ≥ 3%, or does not achieve a 75% improvement in PASI after using a biological agent.

9. The method according to any one of claims 1-8, wherein the anti-IL23p19 antibody is administered to a subject who has lost response or exhibited poor response to previous biological therapy, by a two-stage administration mode comprising a treatment stage and a maintenance stage.

10. The method according to claim 9, wherein during the treatment phase, the anti-IL23p19 antibody is administered at a frequency of once every week, once every two weeks, once every four weeks, once every five weeks, once every six weeks, once every seven weeks, once every eight weeks, or once every twelve weeks.

11. The method according to claim 9 or 10, wherein the treatment phase comprises administering the anti-IL-23p19 antibody at weeks 0, 4, and 8.

12. The method according to any one of claims 9-11, wherein during the maintenance phase, the anti-IL-23p19 antibody is administered at a frequency of once every four weeks, once every eight weeks, once every twelve weeks, once every sixteen weeks, once every twenty weeks, or longer, for at least 24, 28, 32, 36, 40, 44, 48 weeks, or longer.

13. The method according to any one of claims 9-12, wherein, during the maintenance phase, the anti-IL-23p19 antibody is administered once every 12 weeks until the last administration at Week 32.

14. The method according to any one of claims 1-13, wherein the anti-IL-23p19 antibody is administered to a subject who have lost response or exhibited poor response to previous biological therapy at weeks 0, 4 and 8, and administered once every 12 weeks thereafter, for example, the anti-IL-23p19 antibody is administered at weeks 0, 4 and 8, and then administered once every 12 weeks until the last dosage at week 32; for another example, the anti-IL-23p19 antibody is administered at 200 mg at weeks 0, 4 and 8, and then administered at 200 mg once every 12 weeks until the last dosage at week 32.

15. The method according to any one of claims 1-6, wherein the subject is responsive to previous treatment with a biological agent, or the subject is being treated with a biological agent and still responds.

16. The method according to claim 15, wherein the subject has a sPGA score of 0 or 1 and a cumulative body surface area of skin lesions of < 3%, or achieves a PASI improvement of 75% or more after using a biological agent.

17. The method according to any one of claims 15-16, wherein the anti-IL-23pl9 antibody is administered at week 0.

18. The method according to any one of claims 15-17, wherein the anti-IL23p19 antibody is administered at a frequency of once every four weeks, once every eight weeks, once every twelve weeks, once every sixteen weeks, once every twenty weeks, or longer.

19. The method according to any one of claims 15-18, wherein the anti-IL-23p19 antibody is administered at week 0 and once every 12 weeks thereafter.

20. The method according to any one of claims 15-19, wherein the anti-IL-23p19 antibody is administered to a subject who has responded to previous biological therapy at week 0 and then once every 12 weeks for at least 24, 28, 32, 36, 40, 44, 48 weeks or longer.

21. The method according to any one of claims 1-20, wherein the dosage of the anti-IL-23p19 antibody administered each time is selected from 100 mg-1000 mg, preferably 150 mg-800 mg, e.g., 100 mg, 110 mg, 120 mg, 130 mg, 140 mg, 150 mg, 160 mg, 170 mg, 180 mg, 190 mg, 200 mg, 210 mg, 220 mg, 230 mg, 240 mg, 250 mg, 260 mg, 270 mg, 280 mg, 290 mg, 300 mg, 350 mg, 400 mg, 450 mg, 500 mg, 550 mg, 600 mg, 650 mg, 700 mg, 750 mg, 800 mg, 850 mg, 900 mg, 950 mg, or 1000 mg, preferably 200 mg per dose.

22. The method according to any one of claims 1-21, wherein the biological agent comprises a tumor necrosis factor $\alpha$ inhibitors/antagonists and/or an IL-17A inhibitors/antagonists.

23. The method according to claim 22, wherein the TNF-$\alpha$ inhibitors/antagonists comprises etanercept, infliximab, adalimumab, golimumab, and trastuzumab.

24. The method according to claim 22, wherein the IL-17A inhibitors/antagonists comprises secukinumab and ixekizumab.

25. The method according to any one of claims 1-24, wherein the anti-IL-23p19 antibody is formulated into a liquid pharmaceutical composition for administration.

26. The method of claim 25, wherein the liquid pharmaceutical composition is an injection.

27. The method according to any one of claims 1-26, wherein the anti-IL-23p19 antibody or the pharmaceutical composition is administered by subcutaneous injection.

28. Use of an anti-IL-23p19 antibody or a pharmaceutical composition comprising the anti-IL-23p19 antibody in the preparation of a medicament for treating plaque psoriasis in a subject previously treated with a biological agent for plaque psoriasis, wherein the anti-IL-23p19 antibody is as described in any one of claims 1-3.

29. The use according to claim 28, wherein the subject is as described in any one of claims 4-8 and calims 15-16.

30. A single pharmaceutical dosage unit, comprising the anti-IL-23p19 antibody according to any one of claims 1-3.

31. The single pharmaceutical dosage unit according to claim 30, wherein the single pharmaceutical dosage unit comprises the following dosage of the anti-IL-23p19 antibody: a fixed dosage of 10-1000 mg, preferably a fixed dosage of 50-800 mg, more preferably a fixed dosage of 50-500 mg, e.g., 50 mg, 60 mg, 70 mg, 80 mg, 90 mg, 100 mg, 110 mg, 120 mg, 130 mg, 140 mg, 150 mg, 160 mg, 170 mg, 180 mg, 190 mg, 200 mg, 210 mg, 220 mg, 230 mg, 240 mg, 250 mg, 260 mg, 270 mg, 280 mg, 290 mg, 300 mg, 350 mg, 400 mg, 450 mg, 500 mg, 550 mg, 600 mg, 650 mg, 700 mg, 750 mg, 800 mg, 850 mg, 900 mg, 950 mg, or 1000 mg, preferably 50 mg, 60 mg, 70 mg, 80 mg, 90 mg, 100 mg, 150 mg, 200 mg, 300 mg, 350 mg, 400 mg, or 500 mg.

32. The single pharmaceutical dosage unit according to claim 30 or 31, wherein the single pharmaceutical dosage unit is a single dosage unit packaged in a prefilled automatic injection pen.

33. A pharmaceutical kit, comprising the single pharmaceutical dosage unit according to any one of claims 30-32, and optionally, a package insert printed with instructions for use of an anti-IL-23p19 antibody in preventing or treating plaque psoriasis in a patient.

34. Use of the single pharmaceutical dosage unit according to any one of claims 30-32 or the pharmaceutical kit according to claim 33 in the manufacture of a medicament for treating plaque psoriasis.

35. The single pharmaceutical dosage unit according to any one of claims 30-32 or the pharmaceutical kit according to claim 33 for use in treating plaque psoriasis.

36. The use according to claim 34, or the single pharmaceutical dosage unit or the pharmaceutical kit for use according to claim 35, for use in a subject according to any one of claims 4-8 and 15-16.

## INTERNATIONAL SEARCH REPORT

| International application No. |
| --- |
| **PCT/CN2024/110996** |

**A. CLASSIFICATION OF SUBJECT MATTER**

C07K16/24(2006.01)i; C12N15/13(2006.01)i; A61K39/395(2006.01)i; A61P37/00(2006.01)i

According to International Patent Classification (IPC) or to both national classification and IPC

**B. FIELDS SEARCHED**

Minimum documentation searched (classification system followed by classification symbols)

IPC: C07K, C12N, A61K, A61P

Documentation searched other than minimum documentation to the extent that such documents are included in the fields searched

Electronic data base consulted during the international search (name of data base and, where practicable, search terms used)

DWPI, VEN, CNABS, CNTXT, CNKI, PubMed, GenBank, EBI, STN, WPABSC, WPABS, ENTXT, ENTXTC, Wanfang Database, ISI web of Knowledge: IL-23, IL23, IL12, IL-12, IL-23p19, 亚基, 银屑, 斑块, psoriasis, psoriatic arthritis, IL-23p40, GM-CSF, Th1, Th7, 抗体, 给药, SEQ ID NOs: 1-10, 信达

**C. DOCUMENTS CONSIDERED TO BE RELEVANT**

| Category* | Citation of document, with indication, where appropriate, of the relevant passages | Relevant to claim No. |
| --- | --- | --- |
| X | WO 2020108530 A1 (INNOVENT BIOLOGICS (SUZHOU) CO., LTD.) 04 June 2020 (2020-06-04) claims 1-23, and description, embodiments | 1-36 |
| Y | WO 2020108530 A1 (INNOVENT BIOLOGICS (SUZHOU) CO., LTD.) 04 June 2020 (2020-06-04) claims 1-23, and description, embodiments | 1-36 |
| Y | WO 2022190034 A1 (JANSSEN BIOTECH, INC.) 15 September 2022 (2022-09-15) claims 1-30 | 1-36 |
| Y | CN 113825768 A (SUN PHARMACEUTICAL INDUSTRIES LIMITED) 21 December 2021 (2021-12-21) claims 1-44 | 1-36 |
| Y | CN 104507497 A (BOEHRINGER INGELHEIM INTERNATIONAL GMBH) 08 April 2015 (2015-04-08) claims 5-14 and 19-30 | 1-36 |

☑ Further documents are listed in the continuation of Box C. ☑ See patent family annex.

| | |
| --- | --- |
| * Special categories of cited documents:<br>"A" document defining the general state of the art which is not considered to be of particular relevance<br>"D" document cited by the applicant in the international application<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
| --- | --- |
| **11 September 2024** | **24 October 2024** |

| Name and mailing address of the ISA/CN | Authorized officer |
| --- | --- |
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

**EP 4 759 828 A1**

## INTERNATIONAL SEARCH REPORT

| International application No. |
| --- |
| **PCT/CN2024/110996** |

**C.    DOCUMENTS CONSIDERED TO BE RELEVANT**

| Category* | Citation of document, with indication, where appropriate, of the relevant passages | Relevant to claim No. |
| --- | --- | --- |
| Y | WO 2023144699 A1 (SUN PHARMACEUTICAL INDUSTRIES LIMITED) 03 August 2023 (2023-08-03) <br> claims 1-29 | 1-36 |
| Y | CN 103396489 A (MERCK SHARP & DOHME CORP.) 20 November 2013 (2013-11-20) <br> claims 1-10 | 1-36 |
| Y | US 2021188961 A1 (NOVAROCK BIOTHERAPEUTICS, LTD.) 24 June 2021 (2021-06-24) <br> description, embodiments | 1-36 |
| A | US 2022073603 A1 (JANSSEN BIOTECH, INC.) 10 March 2022 (2022-03-10) <br> claims 1-5 | 1-36 |

Form PCT/ISA/210 (second sheet) (July 2022)

## INTERNATIONAL SEARCH REPORT

International application No.

**PCT/CN2024/110996**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

    a. ☑ forming part of the international application as filed.

    b. ☐ furnished subsequent to the international filing date for the purposes of international search (Rule 13*ter*.1(a)),

        ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

## INTERNATIONAL SEARCH REPORT

| International application No. |
|---|
| **PCT/CN2024/110996** |

| **Box No. II** | **Observations where certain claims were found unsearchable (Continuation of item 2 of first sheet)** |
|---|---|

This international search report has not been established in respect of certain claims under Article 17(2)(a) for the following reasons:

1. ☑ Claims Nos.: **1-27**
   because they relate to subject matter not required to be searched by this Authority, namely:

   Claims 1-27 relate to a method for treating plaque-like psoriasis in a subject, that is, claims 1-27 relate to subject matter for which no search is required by the International Searching Authority as defined in PCT Rule 39.1: (4) methods for treatment of the human or animal body by surgery or therapy, as well as diagnostic methods. The international search is performed on the basis of the use of the anti-IL23p19 antibody of claims 1-27 in the preparation of a drug for treating plaque-like psoriasis.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.

**PCT/CN2024/110996**

| Patent document cited in search report | | | Publication date (day/month/year) | Patent family member(s) | | | Publication date (day/month/year) |
|---|---|---|---|---|---|---|---|
| WO | 2020108530 | A1 | 04 June 2020 | None | | | |
| WO | 2022190034 | A1 | 15 September 2022 | None | | | |
| CN | 113825768 | A | 21 December 2021 | IL | 287213 | A | 01 December 2021 |
| | | | | AU | 2020259375 | A1 | 28 October 2021 |
| | | | | BR | 112021020612 | A2 | 28 December 2021 |
| | | | | US | 2022298233 | A1 | 22 September 2022 |
| | | | | CA | 3143604 | A1 | 22 October 2020 |
| | | | | JOP | 20210279 | A1 | 30 January 2023 |
| | | | | EP | 3956357 | A1 | 23 February 2022 |
| | | | | EP | 3956357 | A4 | 04 January 2023 |
| | | | | WO | 2020212874 | A1 | 22 October 2020 |
| | | | | SG | 11202111056 | YA | 29 November 2021 |
| | | | | MA | 55729 | A | 23 February 2022 |
| | | | | KR | 20210140780 | A | 23 November 2021 |
| | | | | MX | 2021012652 | A | 24 January 2022 |
| | | | | JP | 2022529266 | A | 20 June 2022 |
| CN | 104507497 | A | 08 April 2015 | US | 2014178401 | A1 | 26 June 2014 |
| | | | | US | 11078265 | B2 | 03 August 2021 |
| | | | | NZ | 700802 | A | 30 June 2017 |
| | | | | AR | 090923 | A1 | 17 December 2014 |
| | | | | EP | 2844284 | A1 | 11 March 2015 |
| | | | | MX | 2014013149 | A | 19 January 2015 |
| | | | | MX | 368653 | B | 10 October 2019 |
| | | | | PL | 3326649 | T3 | 25 April 2022 |
| | | | | TW | 201840335 | A | 16 November 2018 |
| | | | | TWI | 683669 | B | 01 February 2020 |
| | | | | PH | 12014502406 | A1 | 12 January 2015 |
| | | | | JP | 2015517465 | A | 22 June 2015 |
| | | | | JP | 6293120 | B2 | 14 March 2018 |
| | | | | CL | 2014002954 | A1 | 06 February 2015 |
| | | | | WO | 2013165791 | A1 | 07 November 2013 |
| | | | | EP | 4039275 | A1 | 10 August 2022 |
| | | | | AU | 2013256724 | A1 | 30 October 2014 |
| | | | | ES | 2908474 | T3 | 29 April 2022 |
| | | | | EP | 3326649 | A1 | 30 May 2018 |
| | | | | EP | 3326649 | B1 | 09 February 2022 |
| | | | | UY | 34779 | A | 29 November 2013 |
| | | | | CA | 2871985 | A1 | 07 November 2013 |
| | | | | CA | 2871985 | C | 10 October 2023 |
| | | | | KR | 20150013651 | A | 05 February 2015 |
| | | | | KR | 102124758 | B1 | 19 June 2020 |
| | | | | AU | 2018200239 | A1 | 01 February 2018 |
| | | | | AU | 2018200239 | B2 | 17 October 2019 |
| | | | | BR | 112014027372 | A2 | 08 August 2017 |
| | | | | TW | 201406391 | A | 16 February 2014 |
| | | | | TWI | 631957 | B | 11 August 2018 |
| | | | | EA | 201401204 | A1 | 29 May 2015 |
| | | | | IL | 264558 | A | 28 February 2019 |
| | | | | US | 2021317201 | A1 | 14 October 2021 |
| | | | | IL | 235059 | A0 | 31 December 2014 |

Form PCT/ISA/210 (patent family annex) (July 2022)

## INTERNATIONAL SEARCH REPORT
### Information on patent family members

International application No.

**PCT/CN2024/110996**

| Patent document cited in search report | | | Publication date (day/month/year) | Patent family member(s) | | | Publication date (day/month/year) |
|---|---|---|---|---|---|---|---|
| | | | | IL | 258940 | A | 28 June 2018 |
| | | | | IL | 258940 | B | 30 May 2019 |
| WO | 2023144699 | A1 | 03 August 2023 | AU | 2023211042 | A1 | 08 August 2024 |
| | | | | AU | 2023211042 | A9 | 15 August 2024 |
| | | | | US | 2023303678 | A1 | 28 September 2023 |
| CN | 103396489 | A | 20 November 2013 | MX | 2009009080 | A | 02 November 2009 |
| | | | | US | 2013039916 | A1 | 14 February 2013 |
| | | | | US | 8513389 | B2 | 20 August 2013 |
| | | | | EP | 2064242 | A1 | 03 June 2009 |
| | | | | EP | 2426145 | A1 | 07 March 2012 |
| | | | | EP | 2426145 | B1 | 18 January 2017 |
| | | | | CA | 2678863 | A1 | 28 August 2008 |
| | | | | JP | 2010518858 | A | 03 June 2010 |
| | | | | WO | 2008103473 | A1 | 28 August 2008 |
| | | | | SG | 178804 | A1 | 29 March 2012 |
| | | | | US | 2010111966 | A1 | 06 May 2010 |
| US | 2021188961 | A1 | 24 June 2021 | AU | 2020409124 | A1 | 30 June 2022 |
| | | | | EP | 4077378 | A1 | 26 October 2022 |
| | | | | WO | 2021126435 | A1 | 24 June 2021 |
| | | | | US | 11396541 | B2 | 26 July 2022 |
| | | | | US | 2022332811 | A1 | 20 October 2022 |
| | | | | KR | 20220143005 | A | 24 October 2022 |
| | | | | CA | 3164996 | A1 | 14 June 2021 |
| | | | | JP | 2023509373 | A | 08 March 2023 |
| US | 2022073603 | A1 | 10 March 2022 | WO | 2022024065 | A1 | 03 February 2022 |
| | | | | AU | 2021315081 | A1 | 30 March 2023 |
| | | | | US | 2024101662 | A1 | 28 March 2024 |
| | | | | CA | 3190230 | A1 | 03 February 2022 |
| | | | | EP | 4188443 | A1 | 07 June 2023 |

Form PCT/ISA/210 (patent family annex) (July 2022)

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 2019121261 W

- CN 2021093219 W

**Non-patent literature cited in the description**

- **OPPMANN et al.** *Immunity*, 2000, vol. 13, 715
- **HEATH**. *EMBO J.*, 2000, vol. 19, 2399-2411
- **CHOTHIA et al.** *Nature*, 1989, vol. 342, 877-883
- **AL-LAZIKANI et al.** Standard conformations for the canonical structures of immunoglobulins. *Journal of Molecular Biology*, 1997, vol. 273, 927-948
- **KABAT et al.** Sequences of Proteins of Immunological Interest. U.S. Department of Health and Human Services, 1987
- **KABAT et al.** Sequences of Proteins of Immunological Interest. Public Health Service, National Institutes of Health, 1991